# EUROPEAN PATENT SPECIFICATION

(11) **EP 3 490 427 B1**
(45) Date of publication and mention of the grant of the patent: **12.01.2022**
(21) Application number: 17836516.9
(22) Date of filing: 21.02.2017
(51) Int. Cl.: A61B 1/005

(54) **LARYNGOSCOPY DEVICES**
LARYNGOSKOPVORRICHTUNGEN
DISPOSITIFS DE LARYNGOSCOPIE

(30) Priority: 01.08.2016 IL 24704916
(43) Date of publication of application: 05.06.2019
(73) Proprietor: Benuri-Silbiger, Ishay, 9710657 Jerusalem (IL); Millis, Shahar, 3752071 Pardes Hanna Karkur (IL)
(72) Inventor: BENURI-SILBIGER, Ishay, 9710657 Jerusalem (IL)
(74) Representative: AWA Sweden AB
(86) International application number: PCT/IL2017/050215
(87) International publication number: WO 2018/025255

(56) References cited:
- WO-A1-94/14368
- WO-A1-98/26706
- WO-A1-2011/150469
- CN-Y- 201 005 671
- NL-C2- 1 019 263
- US-A1- 2014 128 681

## Description

### TECHNICAL FIELD

The present disclosure generally relates to devices and methods for airway management, particularly in laryngoscopy.

### BACKGROUND

Endotracheal intubation (ETI) is a medical procedure wherein an endotracheal tube is inserted through a subject's mouth or nose into their trachea. ETI is applied across a vast array of age-groups and disease-states to allow oxygen delivery to the lungs in subjects not capable of breathing on their own, in subjects requiring sedation (such as subjects undergoing surgery), in unconscious subjects at risk of aspirating gastric content, and in subjects at risk of airway obstruction (such as subjects suffering from an allergic reaction or burn victims).

Oral ETI is usually performed by positioning the subject in a "sniffing position" (by extending their head and flexing their neck) and introducing through the mouth a laryngoscope blade (typically a Macintosh blade). The blade slides on the subject's tongue until the distal end of the blade reaches the epiglottic vallecula - a depression at the base of the tongue. The laryngoscope's handle is then pulled, lifting the tongue at its base and bringing into alignment the oral axis with the pharyngeal axis, thereby facilitating an unobstructed view of the glottis and creating a pathway for the endotracheal tube.

A variety of causes may complicate ETI, preventing a quick execution thereof. These include: morbid obesity, inability or contraindication to moving the subject's head (e.g. when cervical spine injury is suspected, thereby preventing positioning the subject in the sniffing position), profound oral secretion or perfuse hemorrhage in the oropharyngeal cavity, airway swelling (e.g. due to an allergic reaction or in burn victims), as well as an often steep learning curve experienced by practitioners.

To facilitate rapid ETI in such cases, several aids have been developed. Currently, perhaps the most widely used aid is the elastic endotracheal introducer - the so-called bougie introducer. The bougie introducer is a long stylet with a bent distal tip. The angle of the distal tip acts to facilitate entering the bougie introducer into the laryngeal inlet. When the bougie introducer has been inserted into the laryngeal inlet of the subject, an endotracheal tube is mounted on the bougie introducer and advanced thereon into the trachea, and the bougie introducer is pulled out.

The bougie introducer provides an effective tool for aiding intubation when the epiglottis is visible, even if the vocal cords are not (Grade III Cormack-Lehane view). However, the bougie introducer is unlikely to be of utility when none of the airway is visible (Grade IV Cormack-Lehane view) for reasons elaborated on below.

According to a recent study, a substantial percentage of bougie introduction attempts result in failure. See, for example, http://felipeairway.sites.medinfo.ufl.edu/files/2009/06/the-american-journal-of-emergency-medicine-2005-jabre.pdf. One of the main reasons for bougie introduction failure is esophageal intubation, that is to say, the insertion of the bougie introducer into the esophagus (instead of into the laryngeal inlet). Being narrow, elongated, and flexible, control of the bougie introducer at its tip can be very challenging, particularly when the tongue base obstructs the path into the trachea.

US patent no. 8,702,599 to De Domenico discloses an improved laryngoscope that is useful in endotracheal intubation. The laryngoscope includes an inner magnetic element that is situated at an end of the laryngoscope's blade and an outer magnetic element that is positioned on a patient's throat in such a manner that the interaction between the inner and outer elements attracts the end of the blade toward the patient's epiglottic vallecula when the blade is moved into the patient's throat. The blade also includes a magnetic bed located along its longitudinal axis. The magnetic bed is designed to interact with a metallic spiral tube and, by means of this interaction, guide the tube properly in the patient's inner air tract. The blade also includes at least three coplanar elements that able to rotate relative to each other. The adjustment of these elements increases the blade's usefulness for moving the patient's tissues and opening a passage to the patient's trachea.

US patent no. 8,366,612 to Rosenthal discloses a method of using a laryngoscope guide including a guiding conduit for advancing an introducer. The method can be used to intubate a subject by positioning the laryngoscope guide in an airway; advancing the introducer in the conduit until an end of the introducer enters the glottis, while viewing images of the introducer end; removing the laryngoscope guide with the guide conduit from the introducer and from the airway; guiding a tube with the introducer until an end of the tube enters the glottis; and removing the introducer from the tube with the end of the tube remaining in the glottis, and in particular, in the trachea, to establish an airway. The guide and method can enable a healthcare professional to intubate a subject where neck mobility is an issue, where an airway is difficult, and/or where a subject is obese.

Some related prior art technologies are disclosed in US 2014/128681 A1, which discloses a laryngoscopy device according to the preamble of claim 1, in NL 1 019 263 C2 and in WO 94/14368 A1.

### SUMMARY

Aspects of the disclosure, in some embodiments thereof, relate to devices and methods for controlling and maneuvering instruments in airway management. More specifically, aspects of the disclosure, in some embodiments thereof, relate to devices and methods for laryngoscopy.

As explained above, there is a need for ETI devices and methods facilitating rapid and accurate ETI. Specifically, there is a need for ETI devices and methods facilitating rapid and accurate ETI in Grade III and particularly Grade IV Cormack-Lehane view conditions. The present application, according to some of the embodiments disclosed herein, addresses this need.

According to an aspect of some embodiments, there is provided a laryngoscopy device including:
- a laryngoscope blade, being elongated, having a blade distal portion, a blade first edge, and a blade second edge, opposite to the blade first edge; and
- a looped element, switchable at least from a first configuration to a second configuration.

In the first configuration, and in the second configuration, the looped element defines an expanded arch and a contracted arch, respectively. The expanded arch has an expanded arch first arm and an expanded arch second arm, joined at an expanded arch top. The contracted arch has a contracted arch first arm and a contracted arch second arm, joined at a contracted arch top.

The expanded arch top is positioned superiorly relative to the blade distal portion. The expanded arch first arm at least partially extends laterally from about the blade first edge and the expanded arch second arm at least partially extends laterally from about the blade second edge.

According to some embodiments, the looped element is continuously switchable from the first configuration to the second configuration, across a range of mid-configurations, defining a respective range of increasingly smaller arches, ranging from the expanded arch to the contracted arch.

According to some embodiments, the looped element is additionally switchable from the second configuration to the first configuration.

According to some embodiments, the looped element is continuously switchable from the second configuration to the first configuration.

According to some embodiments, the contracted arch top is positioned superiorly relative to the blade distal portion.

According to some embodiments, the blade distal portion is curved and the expanded arch and the contracted arch are each configured such as (i) to encompass the epiglottis of a subject when the laryngoscopy device is inserted into the oral cavity of the subject with the blade distal portion being inserted into the vallecula of the subject, and (ii) to simultaneously facilitate guiding of an elongated intubation member through the expanded arch and through the contracted arch, respectively.

According to some embodiments, the elongated intubation member is a bougie introducer, a stylet, or an endotracheal tube.

According to some embodiments, there is provided a laryngoscope including the laryngoscopy blade and a handle, wherein the laryngoscope blade is mechanically connected to the handle.

According to an aspect of some embodiments, there is provided a laryngoscopy device including:
- a laryngoscope blade, being elongated, having a blade distal portion;
- a looped element, switchable at least from a first configuration to a second configuration; and
- a mounting mechanism.

In the first configuration and in the second configuration the looped element respectively defines an expanded arch, having an expanded arch base, and a contracted arch, having a contracted arch base.

The mounting mechanism directly or indirectly supports (for example, mechanically supports, magnetically supports, or both) the expanded arch and the contracted arch in the first configuration and in the second configuration, respectively.

The mounting mechanism substantially restricts the expanded arch base and the contracted arch base to a mounting site on the blade distal portion.

According to some embodiments, an arch top of the expanded arch is positioned superiorly relative to the blade distal portion.

According to some embodiments, the expanded arch base and the contracted arch base are controllably shiftable from the mounting site to at least one other mounting site on the blade distal portion.

According to some embodiments, the expanded arch is wider than the blade distal portion.

According to an aspect of some embodiments, there is provided a method for performing laryngoscopy on a subject. The method includes the steps of:
- providing any one of the laryngoscopy devices and an elongated intubation member;
- introducing the laryngoscopy device into the oral cavity of the subject with the looped element being in the first configuration;
- maneuvering the laryngoscope blade such as to insert the blade distal portion into the vallecula of the subject, thereby encompassing the epiglottis of the subject with the expanded arch;
- introducing the elongated intubation member into the oral cavity and distally advancing the elongated intubation member until a member distal tip thereof passes through the expanded arch and is proximally positioned relative to the corniculate tubercle of the subject;
- switching from the first configuration to the second configuration thereby directing the member distal tip towards the laryngeal inlet of the subject; and
- distally advancing the member distal tip into the laryngeal inlet of the subject.

According to some embodiments, the elongated intubation member is a bougie introducer, and the method further includes, following the step of distally advancing the member distal tip into the laryngeal inlet of the subject, a step of mounting an endotracheal tube on the bougie introducer.

According to some embodiments, the elongated intubation member is an endotracheal tube.

Certain embodiments of the present disclosure may include some, all, or none of the above advantages. One or more technical advantages may be readily apparent to those skilled in the art from the figures, descriptions and claims included herein. Moreover, while specific advantages have been enumerated above, various embodiments may include all, some or none of the enumerated advantages.

The invention is defined in independent claim 1 with preferred embodiments thereof being defined in claims 2 - 13.

### BRIEF DESCRIPTION OF THE DRAWINGS

Examples illustrative of embodiments are described below with reference to figures attached hereto. In the figures, identical structures, elements or parts that appear in more than one figure are generally labeled with a same numeral in all the figures in which they appear. Alternatively, elements or parts that appear in more than one figure may be labeled with different numerals in the different figures in which they appear. Dimensions of components and features shown in the figures are generally chosen for convenience and clarity of presentation and are not necessarily shown in scale. The figures are listed below.
**Figure 1** schematically illustrates a laryngoscopy device, including a laryngoscope blade and looped element in a first configuration (solid curve) and in a second configuration (dashed curve), according to some embodiments;
**Figure 2A** schematically illustrates a laryngoscope including the laryngoscopy device of **Fig. 1** and a handle, the laryngoscopy device being partially inserted into the oral cavity of a subject, with the looped element being in the first configuration and encompassing the epiglottis of the subject, and with a bougie introducer inserted through the looped element, according to some embodiments;
**Figure 2B** schematically illustrates the laryngoscope of **Fig. 2A****,** the laryngoscopy device being partially inserted into the oral cavity of the subject, with the looped element being in the second configuration, encompassing the epiglottis of the subject, and directing the bougie introducer towards the laryngeal inlet of the subject, according to some embodiments;
**Figure 3** schematically illustrates a laryngoscopy device, according to some embodiments;
**Figure 4A** schematically illustrates a laryngoscope, according to some embodiments;
**Figure 4B** schematically illustrates a back-view of a hand grip of a guiding assembly of the laryngoscope of **Fig. 4A****,** according to some embodiments;
**Figure 5** schematically illustrates a laryngoscopy device, according to some embodiments.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

As used herein, in accordance with some embodiments, a "laryngoscope blade" may refer to a single piece (integrally formed) blade, as well as to a non-integrally formed blade including, for example, a blade portion and one or more blade extensions mounted thereon or connected thereto. In particular, in accordance with some embodiments, a "laryngoscope blade" may refer to a blade portion and to an extension mounted thereon, for example, on a distal tip of the blade portion.

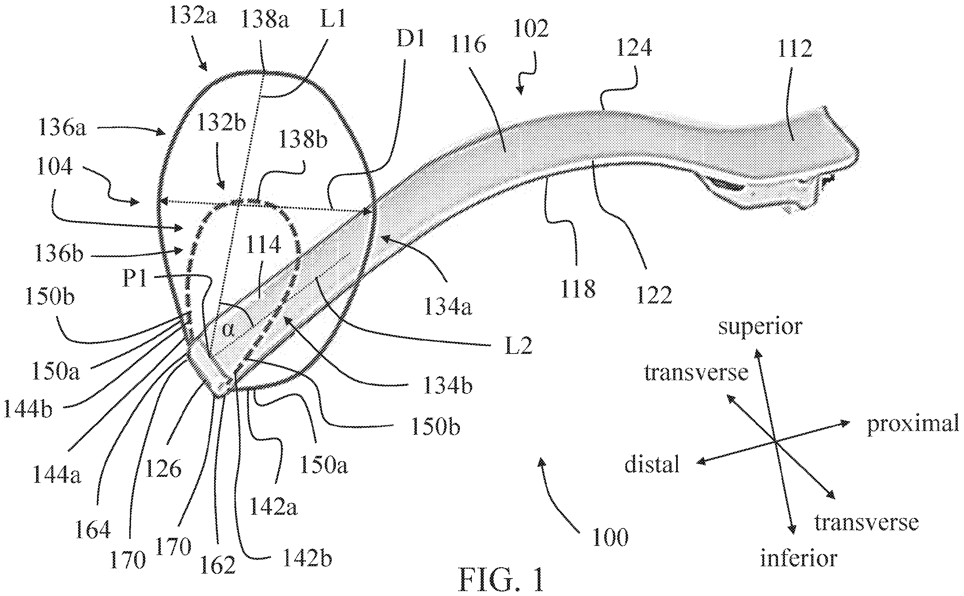

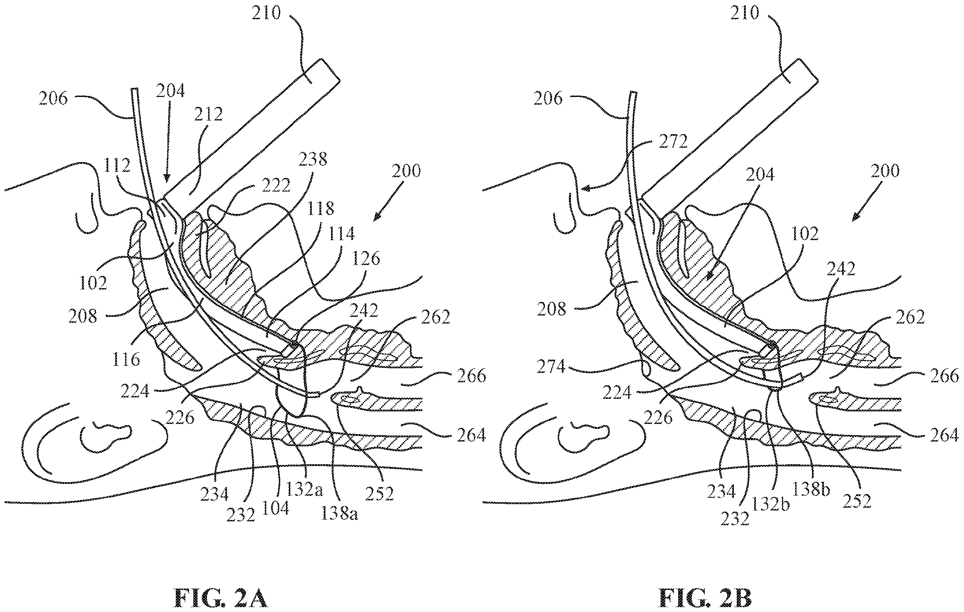

**Fig. 1** schematically depicts a laryngoscopy device **100,** including a laryngoscope blade **102** and a looped element **104,** according to some embodiments of the present disclosure. Laryngoscope blade **102** is elongated and includes a blade proximal portion **112** and a blade distal portion **114.** Laryngoscope blade **102** further includes a blade superior surface **116,** a blade inferior surface **118,** and opposite lateral edges: a blade first edge **122,** and a blade second edge **124.** A blade tip **126** is defined by the distal edge of blade distal portion **114.** According to some embodiments, laryngoscope blade **102** may be mountable on a handle, as depicted, for example, in **Figs. 2A-2B****.**

It is noted that blade first edge **122** and blade second edge **124** need not be symmetric. For example, in some embodiments, blade first edge **122** may be thin, while blade second edge **124** may be thick. In particular, according to some embodiments, blade second edge **124** (or blade first edge **122**) includes a sidewall mounted at right angles to blade superior surface **116** (i.e. perpendicularly to the transverse direction). The sidewall is used to laterally move the tongue during ETI, and is a standard feature in the art of laryngoscopes. For ease of presentation, the sidewall is not depicted in the Figures.

Looped element **104** is switchable from a first configuration to a second configuration. In the first configuration, looped element **104** defines an expanded arch **132a** (marked by a solid curve). In the second configuration looped element **104** defines a contracted arch **132b** (marked by a dashed curve). Expanded arch **132a** extends superiorly relative to blade superior surface **116** at blade distal portion **114.** Expanded arch **132a** includes an expanded arch first arm **134a** and an expanded arch second arm **136a,** which are of substantially equal length. Expanded arch first arm **134a** and expanded arch second arm **136a** are joined at an expanded arch top **138a.**

Expanded arch top **138a** is positioned superiorly relative to blade distal portion **114,** at the most superior position along expanded arch **132a.** An expanded arch first end **142a,** defined by the inferior end of expanded arch first arm **134a,** projects substantially laterally from, or approximately from, blade first edge **122** at blade distal portion **114** (i.e. away from blade distal portion **114**). In some embodiments, expanded arch first end **142a** additionally projects in the superior direction. An expanded arch second end **144a,** defined by the inferior end of expanded arch second arm **136a,** projects substantially laterally from, or approximately from, blade second edge **124** at blade distal portion **114** (i.e. away from blade distal portion **114**). In some embodiments, expanded arch second end **144a** additionally projects in the superior direction.

According to some embodiments, expanded arch **132a** is substantially shaped as a horseshoe, and is mounted substantially at right angles to blade superior surface **116,** with a first and second horse shoe legs mounted approximately at blade first edge **122** and blade second edge **124,** respectively. That is to say, expanded arch first arm **134a** extends laterally and superiorly from blade first edge 122 before starting to curve in the medial direction towards expanded arch top **138a.** Expanded arch second arm **136a** extends laterally and superiorly from blade second edge **124** before starting to curve in the medial direction towards expanded arch top **138a.**

A line **L1** is defined as the straight line extending from expanded arch top **138a** to a point **P1** defined midway between blade first edge **122** and blade second edge **124** at blade tip **126.** A line **L2** is defined as the line extending from point **P1** in the proximal direction along blade superior surface **116** and passing midway between blade first edge **122** and blade second edge **124** at blade distal portion **114.** Lines **L1** and **L2** define an extension angle **α** of expanded arch **132a.** In some embodiments, extension angle **α** is selected to be in the range of about 60° to about 160°, or between about 80° to about 160°, or between about 70° to about 130°, or even between about 75° to about 115°. In some embodiments, extension angle **α** may be controllably adjusted, from about 60° to about 160°, or from about 80° to about 160°, or from about 70° to about 130°, or even from about 75° to about 115°.

Contracted arch **132b** extends superiorly from, or approximately from, blade superior surface **116** at blade distal portion **114.** Contracted arch **132b** includes a contracted arch first arm **134b** and a contracted arch second arm **136b,** which are of substantially equal length. Contracted arch first arm **134b** and contracted arch second arm **136b** are joined at a contracted arch top **138b.** According to some embodiments, contracted arch top **138b** is positioned superiorly relative to blade distal portion **114,** at the most superior position along contracted arch **132b.** According to some embodiments, contracted arch **132b** does not substantially extend superiorly from blade superior surface **116,** substantially extending instead in the distal direction, as elaborated on below. According to some embodiments, contracted arch **132b** is substantially shaped as a horseshoe. Contracted arch **132b** is smaller than expanded arch **132a.**

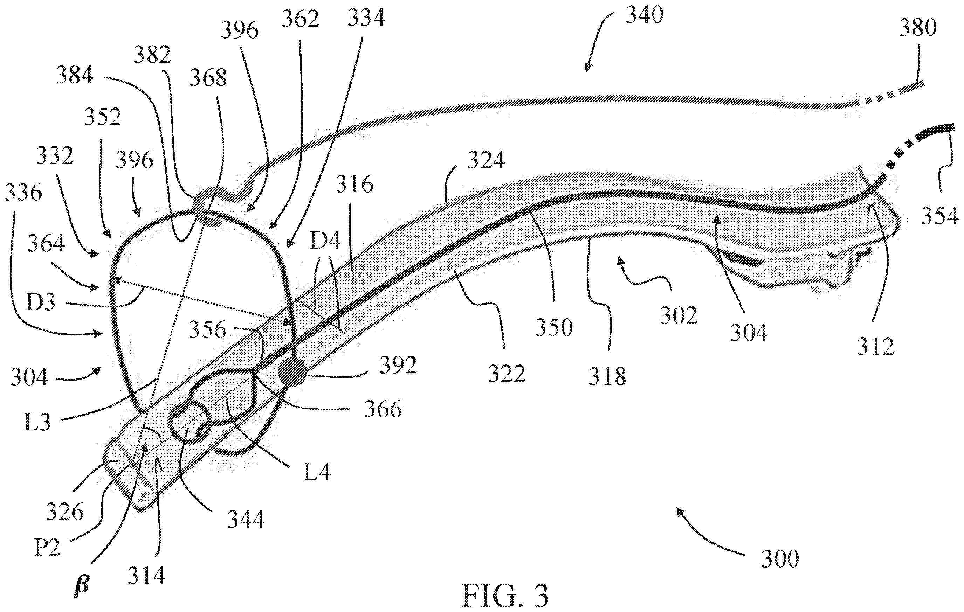

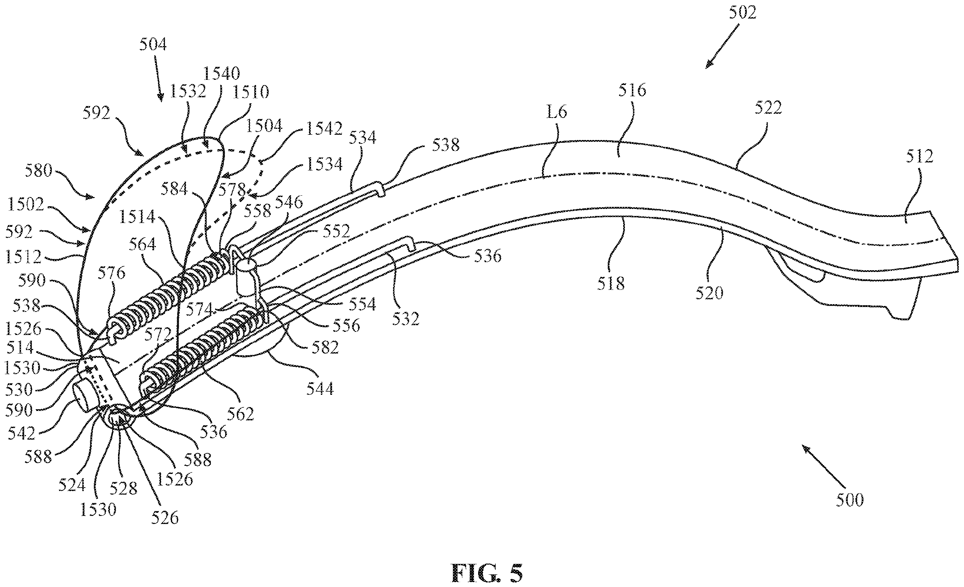

According to some embodiments, looped element **104** is additionally switchable from the second configuration to the first configuration. According to some embodiments, looped element **104** is continuously switchable from the first configuration to the second configuration. That is to say, looped element **104** can be switched across a continuous range of mid-configurations characterized by increasingly smaller arches ranging from expanded arch **132a** to contracted arch **132b.** Similarly, according to some embodiments, looped element **104** is continuously switchable from the second configuration to the first configuration. Mechanisms to achieve and sustain expanded arch **132a** and contracted arch **132b** superior extension, as well as mechanisms for switching from the first configuration to the second configuration, and/or for continuously switching between the configurations, are elaborated on below in the description of **Figs. 3-5****.** According to some embodiments, looped element **104** is pre-shaped, thereby maintaining the superior extension of expanded arch **132a** and contracted arch **132b,** in an essentially similar manner to that described in the description of the laryngoscopy device of **Fig. 5****.** According to some embodiments, looped element **104** is made of flexible and resilient material. For example, looped element **104** may include a stainless steel wire, a wire made of a metal alloy, such as Nitinol, a wire made of flexible polymeric material, such as nylon, or a wire made of a combination of metal and polymeric material. The material can be coated by a non-stick material such as Teflon^{™} to reduce friction. The material can be or include a rare earth magnet to facilitate contact with metal objects (for example a magnetic plate glued to the tip of an endotracheal introducer or tube). In some embodiments, a portion of looped element **104** consisting of expanded arch **132a** may be made of a single stranded wire or a braided wire. In some embodiments the looped element includes chemiluminescence (glow in the dark) material.

In some embodiments, different portions of looped element **104** are made of different materials and have substantially different shapes. For example, in some embodiments the arms of the looped element are made of a plastic wire, while the top of the looped element is made of metal alloy and shaped as a cuboid plate or prism. The arms may be made of a wire that branches in the superior direction to several wires that are connected to a mesh-like structure resembling a rectangular parachute canopy. In some embodiments the canopy angulation can be manipulated by shortening of a single or several of the branched wires. In some embodiments the mesh acts as a net that can entrap and restrict advancement of an object; upon a change in configuration of the object or the net-like structure, the object can be released and advanced further.

In some embodiments, the looped element is composed of separate elements, which are detachable from one another, for example, by mechanical or electrical mechanism, thus allowing to disconnect and pull apart the two arms of an arched formation of the looped element. For example, a grasping forceps commonly used for endoscopic procedures may embody one arm of the looped element, grasping an end of the second arm of the looped element with locked jaws; the resultant arched formation may be dismantled when the locking of the grasper is released and the jaws open. In another example, one arm of the looped element is attached to end of the second arch by an electromagnetic element that can controllably turned off or produce repulsing force. In some embodiments, one arm of the looped element is detached from the base of the arch by similar mechanism (for example, mechanical and/or electrical)

In some embodiments, the looped element may be multi-functional and also be formed of hollow tubing and holes with properties of a suction device. In some embodiments, the looped element includes a mirror that provides the practitioner an additional angle of view of the larynx. In some embodiments, the looped element includes a fiber optic light source, optic fiber transmitting images and/or miniature camera. In some embodiments, the looped element includes elements of electronic sensor or sensors (for example, an infrared or a metal detector) that recognize proximity of an object, enabling blind intubation. In some embodiments, the looped element is made of hollow tubing and an inflatable balloon. Inflation of the balloon may change the arch from one configuration to a more space occupying configuration, obstructing the path to the esophagus and shifting an inserted object toward the larynx

In some embodiments, the arms of the looped element can be placed in the lateral aspect of the blade by parallel conduits running alongside the edges of the blade; another option is that of a single conduit running along the blade and splitting, proximally to the mounting site of the arch, into two branches ('Y' shaped or 'T' shaped) respectively directed (laterally) to the two edges of the blade.

In some embodiments, expanded arch top **138a** may be positioned, for example, at a distance of about 3 cm to about 6 cm from blade distal portion **114.** Expanded arch **132a** may have a width **D1** measuring, for example, between about 2.5 cm to about 4.5 cm. Contracted arch top **138b** may be positioned, for example, at a distance of about 1.5 cm to about 3 cm from blade distal portion **114.** Contracted arch **132b** may have a width (not indicated) measuring, for example, between about 1 cm to about 2 cm.

More specifically, a size of expanded arch **132a** is selected such as to facilitate easy insertion therethrough of a bougie introducer, a stylet, or even an endotracheal tube, when laryngoscopy device **100** is inserted into the oral cavity of a subject, as elaborated on below. A size of contracted arch **132b** is selected such as to direct the bougie introducer, the stylet, and in some embodiments the endotracheal tube, into the laryngeal inlet of the subject, when laryngoscopy device **100** is inserted into the oral cavity of a subject, as elaborated on below. The sizes of expanded arch **132a** and contracted arch **132b** may vary with the size of laryngoscope blade **102.** In particular, the size of expanded arch **132a,** and in some embodiments also the size of second arch **132b,** will depend on physical characteristics of the subject, e.g. height, weight, gender, with the sizes of expanded arch **132a** typically being larger for adults than for children, for adult males than for adult females, etc.

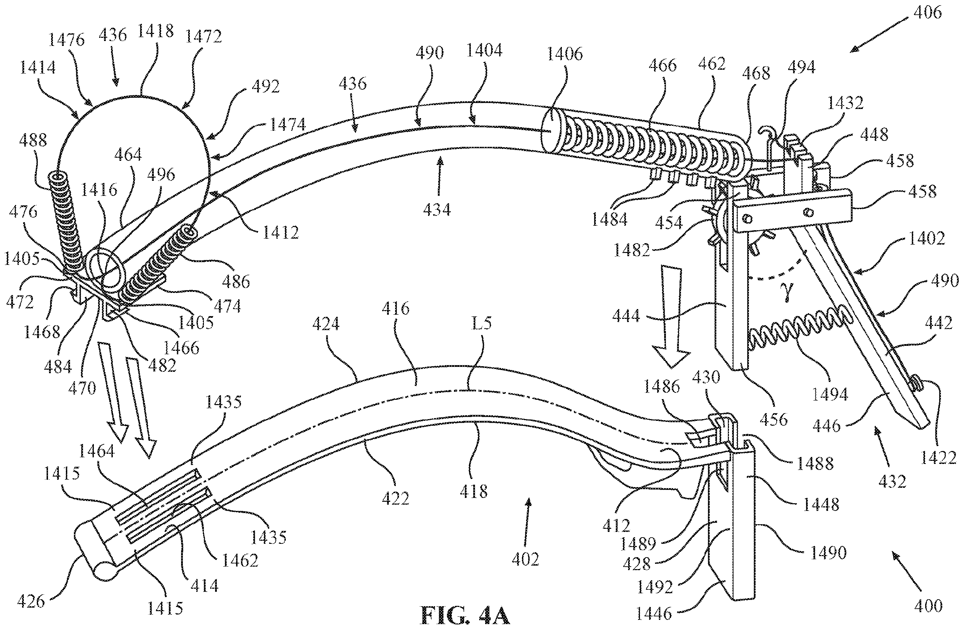

An expanded arch base **150a** is defined by expanded arch first end **142a** and expanded arch second end **144a.** A contracted arch base **150b** is defined by a contracted arch first end **142b** and a contracted arch second end **144b,** that is to say, the inferior ends of contracted arch first arm **134b** and contracted arch second arm **136b,** respectively. In some embodiments, laryngoscopy device **100** includes a mounting mechanism (not shown in **Fig. 1****,** see, for example, **Figs. 4A** and **5**). The mounting mechanism supports arches **132a** and **132b** and further substantially restricts expanded arch base **150a** to a first mounting location **162** on blade first edge **122,** and contracted arch base **150b** to a second mounting location **164** on blade second edge **124.** According to some embodiments, first mounting location **162** and second mounting location **164** are each positioned proximally relative to blade tip **126,** at a distance selected from the range of about 0 cm to about 3 cm from blade tip **126,** or from about 1 cm to about 2.5 cm therefrom. A mounting site **170** consists of mounting locations **162** and **164.**

In some embodiments, arch bases **150a** and **150b** are controllably movable from mounting site **170** to a second mounting site (not shown in **Fig. 1****,** see, for example, **Fig. 4A**) on blade distal portion **114.** The second mounting site is longitudinally positioned (in some embodiments proximally positioned and in some embodiments distally positioned) relative to mounting site **170,** thereby facilitating longitudinally moving expanded arch **132a** in order to accommodate a variety of epiglottic anatomy.

**Figs. 2A-2B** schematically depict laryngoscopy device **100** in operation. **Figs. 2A-2B** depict an embodiment of laryngoscope blade **102** wherein blade distal portion **114** curves in the anterior direction (that is to say, in the inferior direction per the directions defined in **Fig. 1**), similarly to a Macintosh blade. In some embodiments, laryngoscope blade **102** is a Macintosh blade. However, the skilled person will understand that embodiments of laryngoscope blade **102** are not limited to curved blades. In particular, laryngoscope blade **102** may also be embodied as a Miller blade (i.e. with blade distal portion **114** being straight), as elaborated on below.

**Figs. 2A-2B** schematically depict the mouth and upper throat region **200** of a subject with a laryngoscope **204** and a bougie introducer **206** inserted into the oral cavity **208** of the subject. Laryngoscope **204** includes laryngoscopy device **100** and a handle **210.** Blade proximal portion **112** is (mechanically) connected to a handle top **212** of handle **210.** In some embodiments, blade proximal portion **112** is mounted on handle top **212** in an 'r'-like configuration (per the directions defined in **Fig. 1**).

In operation, laryngoscopy device **100** is inserted into oral cavity **208,** with looped element **104** being in the first configuration. Laryngoscope blade **102** is maneuvered such that blade inferior surface **118** is slid on the tongue **222** of the subject, and blade distal portion **114** is inserted into the vallecula **224** of the subject. As blade distal portion **114** is inserted into vallecula **224,** expanded arch **132a** comes to encompass the epiglottis **226** of the subject, with expanded arch top **138a** positioned proximately to, or resting against, the laryngopharyngeal posterior wall **232** (that is to say, the posterior wall of the laryngopharynx **234** of the subject), as shown in **Fig. 2A****.** Handle **210** is then directed anteriorly and caudally (distally) with respect to the subject, thereby lifting the base of the tongue **238** of the subject, and bringing into alignment the oral axis of the subject with their pharyngeal axis (both not indicated), as is standard in the art of laryngoscopy.

Next, bougie introducer **206** is introduced into oral cavity **208** and distally advanced until a distal tip **242** of bougie introducer **206** passes through expanded arch **132a,** such as to be (distally) positioned beyond expanded arch **132a,** for example, by about 1 cm, or by about 2 cm with distal tip **242** being positioned proximally relative to the corniculate tubercle **252** of the of the subject (whereat the laryngeal inlet **262** of the subject and the esophagus **264** of the subject meet).

After distal tip **242** has been advanced through expanded arch **132a,** looped element **104** is switched from the first configuration to the second configuration, which is depicted in **Fig. 2B****.** Contracted arch top **138b** is positioned more proximately to epiglottis **226** than to laryngopharyngeal posterior wall **232.** As bougie introducer **206** is distally advanced farther, contracted arch **132b** directs distal tip **242** into laryngeal inlet **262.** Next an endotracheal tube is mounted on bougie introducer **206** and advanced thereon into laryngeal inlet **262** and therefrom into the trachea **266** of the subject.

In some embodiments, wherein an elongated, large caliber instrument (such as an endotracheal tube) rather than a small caliber instrument (such as a stylet or a bougie introducer, such as bougie introducer **206**) is inserted through contracted arch **132b** (without being mounted on a bougie introducer) contracted arch top **138b** may be positioned more proximately to laryngopharyngeal posterior wall **232** than to epiglottis **226.** In some embodiments, expanded arch **132a** size is sufficiently big to admit insertion therethrough of an endotracheal tube, while contracted arch **132b** may be too small to admit insertion therethrough of an endotracheal tube (but nevertheless sufficiently big to admit insertion therethrough of a bougie introducer, such as bougie introducer **206**).

According to some embodiments, expanded arch **132a** does not substantially extend superiorly from blade superior surface **116,** substantially extending instead in the distal direction. Before inserting the laryngoscope device into the oral cavity, the arch is folded (e.g. manually folded by the person performing the ETI) in the proximal direction (that is to say, the arch top is made to point proximally). During the insertion, the arch will be kept folded, being prevented from unfolding due to the comparably narrow dimensions of the oropharynx and the laryngopharynx. Specifically, so long as the arch is sufficiently long (e.g. about 10 cm) the arch top will rest against the posterior wall of the pharynx. Once the blade tip is inserted into the vallecula, the arch is contracted and the arch top detaches from the posterior wall of the pharynx. The arch top then partially unfolds and comes to encompass the epiglottis. The lateral ligaments of the epiglottis prevent a full unfolding of the arch (that is to say, prevent the arch from returning to the original orientation thereof in the distal direction), thereby leading to an encompassing of the epiglottis with a desired angulation of the arch, through which intubation devices can be inserted.

According to some embodiments, expanded arch **132a** is substantially as wide as blade distal portion **114.** That is to say, expanded arch first arm **134a** and expanded arch second arm **136a** do not laterally project from blade first edge **122** and blade second edge **124,** respectively. According to some embodiments, blade distal portion **114** is wider than expanded arch **132a.**

According to some embodiments, laryngoscopy device **100** may include one or more additional looped elements (not shown) similar to looped element **104.** For instance, beyond looped element **104,** a second looped element (not shown) may be mounted on blade distal portion **114.** The second looped element may be positioned proximally relative to looped element **104.** For example, looped element **104** may be mounted on blade tip **126,** while the second looped element may be mounted on blade distal portion **114,** at a distance (as measured along line **L2**) of about 1 cm or about 2 cm from looped element **104.** Similarly to looped element **104,** the second looped element is also switchable between at least two configurations, which define respective expanded and contracted arches. In particular, the second looped element's expanded arch may be bigger or smaller than expanded arch **132a** and differently shaped and/or oriented. Similarly, the second looped element's contracted arch may be bigger or smaller than contracted arch **132b** and differently shaped and/or oriented.

In some embodiments, the second looped element is associated with the looped element **104.** For example, the second looped element and looped element **104** may be connected by a thin metal net or a thin plastic film. The second looped element may help in guiding bougie introducer **206** (or other types of elongated intubation members, such an endotracheal tube) into laryngeal inlet **262** during ETI, by providing better support and the possibility of angulation (for example, when the second looped element's expanded arch is bigger than expanded arch **132a**).

According to some embodiments, laryngoscope device **100** can be used for nasal ETI. In operation, laryngoscope blade **102** is inserted into oral cavity **208** when looped element **104** is in the first configuration. Laryngoscope blade **102** is maneuvered such that blade inferior surface **118** is slid on the tongue **222** of the subject, and blade distal portion **114** is inserted into the vallecula **224** of the subject. As blade distal portion **114** is inserted into vallecula **224,** expanded arch **132a** comes to encompass the epiglottis **226** of the subject, essentially as described above for oral ETI. A nasal endotracheal tube (not shown) is inserted into a nostril **272** of the subject. The nasal endotracheal tube is distally advanced through the nasal passage (not indicated) until a distal tip thereof exits the nasal passage into the nasopharynx **274** of the subject. The nasal endotracheal tube is distally advanced farther until the distal tip passes through expanded arch **132a** and is positioned proximally relative to conmiculate tubercle **252.** Looped element **104** is then switched from the first configuration to the second configuration, thereby directing the distal tip of the elongated member towards laryngeal inlet **262,** essentially as described above in the description of **Figs. 2A-2B****.**

According to some embodiments, blade distal portion **114** is straight (e.g. laryngoscope blade **102** is a Miller-type blade). In operation, laryngoscope blade **102** is inserted into oral cavity **208** when looped element **104** is in the first configuration. Laryngoscope blade **102** is then maneuvered such that blade inferior portion **118** anteriorly presses against epiglottis **226,** thereby pushing epiglottis **226** against base of the tongue **238.** An elongated member, such as bougie introducer 206, is next inserted into oral cavity **208** and distally advanced until a distal tip thereof passes through expanded arch **132a** and is positioned proximally relative to corniculate tubercle **252.** Looped element **104** is then switched from the first configuration to the second configuration, thereby directing the distal tip towards laryngeal inlet **262,** essentially as described above in the description of **Figs. 2A-2B****.**

**Fig. 3** schematically depicts a laryngoscopy device **300,** including a laryngoscope blade **302,** a looped element **304,** and a flexible rod **340,** according to some embodiments of the present disclosure. Laryngoscope blade **302** is elongated and includes a blade proximal portion **312** and a blade distal portion **314** (per the directions defined in **Fig. 1** which also apply for **Fig. 3**)**.** Laryngoscope blade **302** further includes a blade superior surface **316,** a blade inferior surface **318,** and opposite lateral edges: a blade first edge **322,** and a blade second edge **324.** A blade tip **326** is defined by the distal edge of blade distal portion **314.** Laryngoscope blade **302** is essentially similar to laryngoscope blade **102** and further includes a bore **344.** Bore **344** extends from blade inferior surface **318** to blade superior surface **316** at blade distal portion **314.** According to some embodiments, laryngoscope blade **302** can be mounted on a handle, such as handle **210,** essentially as depicted, for example, in **Figs. 2A-2B****.**

Looped element **304** includes an elongated wire **350** and a loop **352.** Elongated wire **350** extends from a wire proximal end **354** to a wire distal end **356.** Loop **352** includes a loop first arm **362** and a loop second arm **364,** which are of substantially equal lengths. Each of first loop arm **362** and second loop arm **364** extends from a loop base **366** to a loop top **368.** That is to say, loop first arm **362** and loop second arm **364** are joined both at loop base **366** and at loop top **368.** Loop top **368** is positioned superiorly relative to blade distal portion **314,** at the most superior position along loop **352.** Elongated wire **350** and loop **352** are mechanically connected at wire distal end **356** and at loop base **366,** respectively. In some embodiments, elongated wire **350** and loop **352** are integrally formed of a single wire, which is flexible, being shaped into a loop at a distal portion thereof. In some embodiments, loop **352** is made of a different material than elongated wire **350.** For example, elongated wire **350** may be metallic and loop **352** may be made of plastic. Or, for example, elongated wire **350** may be made of one type of metal or alloy and loop **352** may be made of another type of metal or alloy. In some embodiments, loop base **366** is fused onto wire distal end **356.** In some embodiments, loop base **366** is glued onto wire distal end **356.**

Loop **352** includes an arched portion **332.** Arched portion **332** is similar to expanded arch **132a,** being substantially shaped as a horseshoe and extending superiorly relative to blade superior surface **316** at blade distal portion **314.** Arched portion **332** includes an arched portion first arm **334** and an arched portion second arm **336.** Arched portion first arm **334** consists of a portion of loop first arm **362,** which extends from, or approximately from, blade first edge **322** to loop top **368.** Arched portion second arm **336** consists of a portion of loop second arm **364,** which extends from, or approximately from, blade second edge **324** to loop top **368.**

Similarly to looped element **104,** looped element **304** is switchable from a first configuration to a second configuration. In the first configuration, arched portion **332** defines an expanded arch (not shown) similar to expanded arch **132a.** In the second configuration, arched portion **332** defines a contracted arch (not shown) similar to contracted arch **132b.** Further, looped element **304** is continuously switchable from the first configuration to the second configuration, defining a range of mid-configurations with increasingly smaller arches, as elaborated on below. **Fig. 3** depicts one of these mid-configurations (i.e. in **Fig. 3** arched portion **332** is not fully expanded or contracted). According to some embodiments, arched portion **332** is wider than blade distal portion **314.** That is to say, a straight line **D3,** which is defined as the longest line extending in the transverse direction from arched portion first arm **334** to arched portion second arm **336,** is longer than a line **D4,** which is defined as extending in the transverse direction from blade first edge **322** to blade second edge **324** at blade distal portion **314.** According to some embodiments, arched portion **332** is substantially as wide as blade distal portion **314,** or even narrower than blade distal portion **314.**

It is noted that in some embodiments, wherein laryngoscope blade **302** includes a sidewall, as described in the description of laryngoscopy device **100,** and wherein the sidewall distally extends beyond bore **344,** arched portion **332** encompasses the sidewall. In other embodiments, wherein laryngoscope blade **302** includes the sidewall, arched portion second arm **336** extends through a hole in the sidewall; the hole being positioned proximately to blade superior surface **316.**

Flexible rod **340** extends from a rod proximal end **380** to a (distal) rod tip **382.** Looped element **304** and flexible rod **340** are connected at loop top **368** and at rod tip **382,** respectively. Rod tip **382** holds loop **352,** thereby sustaining, or helping to sustain, arched portion **332** superior extension. In some embodiments, loop top **368** is fused onto rod tip **382.** In some embodiments, loop top **368** is glued onto rod tip **382.** In some embodiments, looped element **304** and flexible rod **340** connection is detachable, i.e. looped element **304** and flexible rod **340** can be connected and later disconnected and vice-versa. An example of such a detachable connection is depicted in **Fig. 3****,** wherein rod tip **382** is embodied in the form of a hook **384.** Hook **384** holds loop **352** at loop top **368.** In some embodiments, rod **340** also has properties of an endotracheal introducer. In such embodiment, the rod can be detached from the loop and advanced into the larynx and act as an endotracheal introducer. In some embodiments, hook 384 can be replaced by a circular element that holds loop **352** at loop top **368;** the circular element contains closing mechanism that resembles a circular earrings spring closure. The circular element can be opened or closed by a string that extends from the distal end of the rod/endotracheal introducer. In some embodiments hook **384** can be replaced by an element resembling an endoscopic grasping tool that holds loop **352** at loop top **368.**

A line **L3** is defined by the straight line extending from loop top **368** to a point **P2** defined midway between blade first edge **322** and blade second edge **324** at blade tip **326.** A line **L4** is defined as the line extending from point **P2** in the proximal direction along blade superior surface **316** and passing midway between blade first edge **322** and blade second edge **324** at blade distal portion **314.** Lines **L3** and **L4** define an extension angle **β.** By distally pushing flexible rod **340,** extension angle **β** may be increased (i.e. loop top **368** is distally pushed). By proximally pulling flexible rod **340** (e.g. by proximally pulling on rod proximal end **380**), extension angle **β** may be decreased (i.e. loop top **368** is proximally pulled). In some embodiments, laryngoscopy device **300** includes a string in place of flexible rod **340,** and looped element **304** and the string are connected at loop top **368** and at the distal end of the string, respectively. In such embodiments extension angle **β** may only be decreased (since the string may only be pulled but not pushed).

It is noted that laryngoscopy device **300** can be easily assembled, e.g. by a doctor or a nurse, from laryngoscope blade **302,** looped element **304,** and flexible rod **340.** For example, wire proximal end **354** may be threaded through bore **344** from blade inferior surface **318** to blade superior surface **316.** Wire proximal end **354** may then be proximally pulled, until loop base **366** approaches/reaches/passes bore **344.** Loop **352** is next pulled superiorly and brought over blade tip **326.** Loop **352** is then attached to hook **384** at loop top **368.** In embodiments wherein looped element **304** and flexible rod **340** are integrally formed, laryngoscopy device **300** may be assembled by passing flexible rod through bore **344** from blade superior surface **316** to blade inferior surface **318.** When a sufficiently large portion of loop **352** has been threaded through bore **344,** flexible rod **340** may be maneuvered such as to superiorly pull loop **352** and to bring loop **352** over blade tip **326.**

To switch from the first configuration to the second configuration, elongated wire **350** is pulled, e.g. at wire proximal end **354,** in the proximal direction, thereby contracting arched portion **332.** More specifically, loop base **366** is pulled in the proximal direction, arched portion first arm **362** and arched portion second arm **364** shorten, and loop top **368** is pulled in the inferior direction (causing, in some embodiments, extension angle **β** to decrease). Similarly, looped element **304** can be switched from the first configuration to any one of the mid-configurations, or from one of the mid-configurations to another one defining a smaller arch than the former.

In some embodiments, loop first arm **362** includes a bead **392,** mounted thereon. Bead **392** has a diameter greater than that of bore **344.** Bead **392** is positioned on loop first arm **362** such as to block bore **344** when looped element **304** is in the second configuration, thereby preventing any further pulling of loop **352** through bore **344** and maintaining the second configuration. Further, in some embodiments, bead **392** may rotate about loop first arm **362** at bead **392** mounting position. During ETI, bead **392** rotation may help to decrease friction between loop **352** and an elongated member passed therethrough, such as a thick endotracheal tube. In these embodiments, bead **392** may have a diameter smaller than that of bore **344.** In some embodiments, two or more beads are mounted on loop first arm **362** and/or loop second arm **364.** In some embodiments, a shock-absorbing bead (not shown), that is to say, a bead made of a material with shock absorbent properties, such as rubber, can be mounted on loop **352** at loop top **368.** The shock-absorbing bead, being mounted on a portion of loop **352,** which may induce the most substantial anteriorly directed force on an instrument inserted during ETI (e.g. bougie introducer **206**) as compared to any other portion of loop **352,** may help to prevent abrupt jerking movements of the instrument's tip towards laryngeal inlet **262,** thereby preventing possible damage to surrounding tissue.

In some embodiments, loop **352** is made of a material having little or substantially no flexibility and rigidity, such as a string. In such embodiments, arched portion **332** superior extension may be maintained exclusively by flexible rod **340.**

In some embodiments, loop top **368** consists of a triangularly shaped projection, which may help prevent deformation of loop **352** when inserting laryngoscope blade **302** into oral cavity **208** during ETI.

Laryngoscopy device **300** operation is similar to that of laryngoscope **204.** In operation, laryngoscopy device **300** is inserted into oral cavity **208** with loop top **368** attached onto rod tip **382.** Looped element **304** is in the first configuration, thereby facilitating encompassing of epiglottis **226** by loop **352** as laryngoscope blade **302** is inserted into vallecula **224.** An elongated member is then introduced into oral cavity **208** and distally advanced until a tip thereof passes through loop **352,** reaching a position proximal to corniculate tubercle **252,** essentially as described in the description of **Figs. 2A-2B****.** When the elongated member is thin, e.g. when the elongated member is a bougie introducer, looped element **304** is then switched to the second configuration (i.e. elongated wire **350** is proximally pulled, thereby contracting arched portion **332**). When the elongated member is large caliber, e.g. when the elongated member is an (large caliber) endotracheal tube, instead of being switched to the first configuration, looped element **304** is switched to one of the mid-configurations; the second configuration being too narrow to accommodate the endotracheal tube or easy advancement of the endotracheal tube through the second arch (defined by the second configuration). As the elongated member is distally advanced farther, loop **352** directs the elongated member into laryngeal inlet **262,** essentially as described in the description of **Fig. 2B****.**

During laryngoscope blade **302** insertion into vallecula **224,** extension angle **β** can be adjusted, e.g. by distally pushing or proximally pulling flexible rod **340.** Such adjustment of extension angle **β** may ease encompassing of epiglottis **226** by loop **352.** Extension angle **β** may also be adjusted after looped element **304** has been switched from the first configuration to the second configuration or to one of the mid-configurations. Such adjustment of extension angle **β** may help in guiding the elongated member through loop **352.**

In embodiments wherein elongated wire **350** is rigid, looped element **304** can also be switched back and forth between the mid-configurations and the second configuration. For example, if after switching to the second configuration it is found that the second configuration does not allow for an easy (distal) advancement of the elongated member, looped element **304** may be loosened to a mid-configuration defining a larger arch, and, if need be, subsequently slightly tightened (i.e. switched to a more contracted mid-configuration). Or, for example, switching from the first configuration to the second configuration may be impossible - the elongated member being too thick to allow attaining the second configuration - so that looped element **304** is switched instead to one of the mid-configurations. Looped element **304** may then have to be loosened to allow for an easy advancement of the elongated member.

In some embodiments, loop **352** includes a loop top portion **396** which is made of heavier and less flexible material than a rest of loop **352,** and which is little curved. In some embodiments, loop top portion **396** is substantially straight. Loop top portion **396** includes loop top **368,** in the middle thereof. In some embodiments, loop top portion **396** has a length of about 2.5 cm, of about 3 cm, or even of about 4 cm (and may be wider than blade distal portion **314,** which may have a width of about 2 cm: a Macintosh 4 blade, for example has a width of 1.8 cm), while in the second configuration arched portion **332** has a length of about 4 cm, of about 5 cm, or even of about 6 cm (in the first configuration arched portion **332** has a length of about 9 cm, 10 cm, or even of about 11 cm). When looped element **304** is switched from the first configuration to the second configuration or to one of the mid-configurations, due to loop top portion **396** resilience, loop top portion **396** retains the shape thereof. Consequently, the second arch, or an arch defined by one of the mid-configurations, e.g. arch **332,** is wider than blade distal portion **314,** which may ease insertion of large caliber instruments during ETI. Further, loop top portion **396** may be covered in Teflon^{™}, further easing the insertion of large caliber instruments during ETI by reducing friction between the instruments and loop **352.**

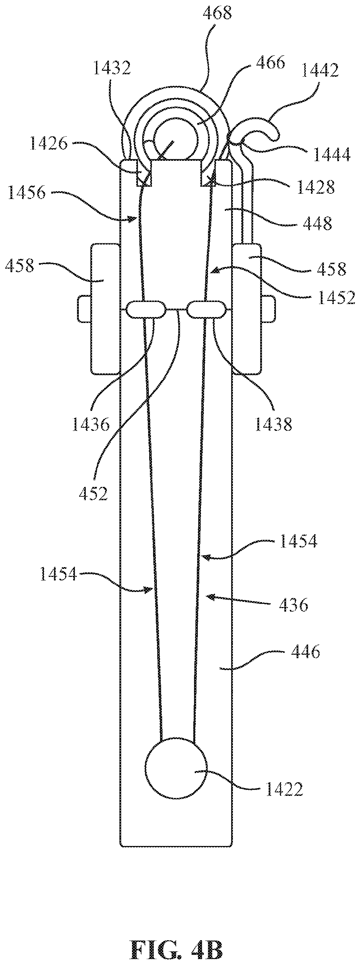

**Fig. 4** schematically depicts a laryngoscope **400,** including a laryngoscope blade **402** and a guiding assembly **406,** according to some embodiments of the present disclosure. Guiding assembly **406** is detachably mountable on laryngoscope blade **402,** as elaborated on below. Laryngoscope blade **402** is elongated and includes a blade proximal portion **412** and a blade distal portion **414.** Laryngoscope blade **402** further includes a blade superior surface **416,** a blade inferior surface **418,** and opposite lateral edges: a blade first edge **422** and a blade second edge **424.** A blade tip **426** is defined by the distal edge of blade distal portion **414.**

A length of laryngoscope blade **402** is indicated by a line **L5,** which extends from the proximal edge (not numbered) of blade proximal portion **412** to blade tip **426,** midway between blade edges **422** and **424.** Laryngoscope blade **402** is essentially similar to laryngoscope blade **102** and further includes an elongated housing **428,** mounted perpendicularly to laryngoscope blade **402** length at the proximal edge of blade proximal portion **402.** Elongated housing **428** extends superiorly from a housing inferior end **1446** to a housing superior end **1448.** Housing superior end **1448** includes a housing slot **430,** extending transversely to line **L5,** and which is level with blade superior surface **416.**

Guiding assembly **406** includes a hand grip **432,** a tubular member **434,** which is elongated, and a looped element **436.** Hand grip **432** includes a handle **442** and a mounting bar **444.** Handle **442** includes a handle elongate portion **446** and a handle superior portion **448** positioned superiorly relative to handle elongate portion **446.** Handle elongate portion **446** and handle superior portion **448** are joined at a handle joint **452** (shown in **Fig. 4B**), with handle superior portion **448** projecting at an angle (not indicated) relative to handle elongate portion **446,** as elaborated on below. Mounting bar **444** includes a mounting bar superior portion **454** and a mounting bar inferior portion **456.** Mounting bar **444** is configured to be slid into elongated housing **428** (via housing slot **430**), as elaborated on below.

Handle **442** and mounting bar **444** are mechanically connected at handle joint **452** and mounting bar superior portion **454,** respectively, through connecting bars **458.** Handle elongate portion **446** and mounting bar **444** subtend a pivot angle **γ.** Handle **442** is configured for a pivoting motion about handle joint **452,** that is to say, a motion which alters pivot angle **y,** as elaborated on below.

Tubular member **434** extends from a member proximal portion **462** to a member distal portion **464.** Tubular member **434** is mounted on mounting bar **444** at member proximal portion **462** and a superior tip (not numbered) of mounting bar superior portion **454,** respectively. In some embodiments member proximal portion **462** is mounted substantially at right angles to mounting bar **444.**

A spring **466** is longitudinally disposed within tubular member **434** at member proximal portion **462.** In some embodiments, spring **466** is a spring coil. In some embodiments, spring **466** is connected at a proximal end thereof (not numbered) to a member proximal tip **468** (that is to say, the proximal edge of member proximal portion **462**). In some embodiments, member proximal tip **468** is walled, except for a hole (not shown) for admitting a wire therethrough, thereby blocking spring **466** at the proximal end thereof.

A connector **470** is attached (e.g. glued) to member distal portion **464** at an inferior portion thereof (not numbered). In some embodiments, tubular member **434** and connector **470** may be integrally formed. Connector **470** is configured to be detachably mounted on blade distal portion **414,** as elaborated on below. Connector **470** includes a connector body **472.** Connector body **472** is elongated and positioned transversely relative to the length of tubular member **434,** transversely extending from a connector first edge **474** to a connector second edge **476.** Connector body **472** further includes a superior surface (not numbered). A connector first leg **482** and a connector second leg **484** project in the inferior direction from an inferior surface (not numbered) of connector body **472.** Connector legs **482** and **484** are configured for snap-in engagement with blade distal portion **414,** as elaborated on below.

A first spring coil **486** and a second spring coil **488** are mounted on the superior surface of connector body **472.** First spring coil **486** is mounted proximately to connector first edge **474** and second spring coil **488** is mounted proximately to connector second edge **476.** First spring coil **486** is mounted such as to extend superiorly relative to connector body **472** and transversely relative thereto (that is to say, side-ways and away from connector body **472**). Similarly, second spring coil **488** is mounted such as to extend superiorly relative to connector body **472** and transversely relative thereto (that is to say, side-ways and away from connector body **472**). A functionality of spring coils **486** and **488** is elaborated on below.

Looped element **436** includes an elongated wire **490** and a loop **492.** Elongated wire **490** extends from a wire proximal end **494** to a wire distal end **496,** and includes a wire proximal portion **1402** and a wire distal portion **1404.** A disc **1406** is disposed within member proximal portion **462,** perpendicularly to the length of tubular member **434** and adjacently to the distal end (not numbered) of spring **466.** Wire proximal portion **1402** includes wire proximal end **494** and is connected on the second end (not numbered) thereof to disc **1406.** Wire distal portion **1404** includes wire distal end **496** and is connected on the second end (not numbered) thereof to disc **1406.** Wire proximal portion **1402** is mechanically associated with handle **442,** as elaborated on below.

Loop **492** includes a loop first arm **1412** and a loop second arm **1414,** each extending from a loop base **1416** to a loop top **1418.** Loop first arm **1412** and loop second arm **1414** are joined at loop top **1418.** Elongated wire **490** and loop **492** are joined at wire distal end **496** and at loop base **1416,** respectively. Loop first arm **1412** and loop second arm **1414** pass through first spring coil **486** and second spring coil **488,** respectively.

**Fig 4B** schematically depicts a back-view of hand grip **432.** Handle elongate portion **446** includes a projecting member **1422.** Projecting member **1422** is shaped substantially as a nail and proximally projects from handle elongate portion **446.** Handle superior portion **448** includes a first notch **1426** and a second notch **1428** on a handle superior portion edge **1432** (that is to say, the superior end of handle superior portion **448**). A first ring **1436** and a second ring **1438** are mounted on handle joint **452,** inferiorly to first notch **1426** and to second notch **1428,** respectively. First ring **1436** mounting is such that a symmetry axis thereof (not shown) points in the superior direction. Similarly, second ring **1438** mounting is such that a symmetry axis thereof (not shown) points in the superior direction.

Wire proximal end **494** is detachably connected to one of connecting bars **458.** For example, one of connecting bars **458** may include a hook **1442** and wire proximal end **494** may include a slip-on ring **1444,** configured to be slipped on hook **1442.** Proximal wire portion **1402** includes three wire segments: a wire first segment **1452,** a wire second segment **1454,** and a wire third segment **1456.** Wire first segment **1452** extends from hook **1442,** passing through second notch **1428,** into second ring **1438.** Wire second segment **1454** extends from second ring **1438** to projecting member **1422,** winds half-way about projecting member **1422,** and extends therefrom into first ring **1436.** Wire third segment **1456** extends from first ring **1436,** via first notch **1426,** into member proximal portion **462** (in embodiments wherein member proximal tip **468** is walled, through the hole in the wall) and thereby onto disc **1406.**

Rings **1436** and **1438** function to ensure that along handle **442** wire proximal portion **1402** extends in parallel to handle **442,** thereby substantially fixing a length of wire proximal portion **1402** which extends from first notch **1426** to second notch **1428,** half-winding along the way around projecting member **1422.** In some embodiments, handle **442** does not include rings **1436** and **1438,** and projecting member 1422 is not mounted on handle elongate portion **446,** being mounted instead on handle superior portion **448.**

In some embodiments, blade distal portion **414** includes a first slot **1462** and a second slot **1464.** Connector first leg **482** and connector second leg **484** include a first rib **1466** and a second rib **1468,** respectively. First rib **1466** forms a protrusion on connector first leg **482,** pointing laterally. Similarly, second rib **1468** forms a protrusion on connector second leg **484,** pointing laterally. First rib **1466** and second rib **1468** are configured to facilitate snap-in engagement between connector first leg **482** and first slot **1462** and between connector second leg **484** and second slot **1464.** In some embodiments, blade distal portion **414** includes a pair of opposite notches (not shown) on blade first edge **420** and blade second edge **422,** respectively. In such embodiments, first rib **1466** points medially and second rib **1468** points medially, thereby facilitating snap-in engagement between connector **470** and blade distal portion **414.**

Guiding assembly **406** can be mounted on laryngoscope blade **402** by sliding mounting bar **444** into elongated housing **428,** via housing slot **430,** and inserting connector first leg **482** and connector second leg **484** into first slot **1462** and second slot **1464,** respectively. When guiding assembly **406** is mounted on laryngoscope blade **402,** tubular member **434** is longitudinally disposed along blade superior surface **416.** In some embodiments, tubular member **434** is flexible and thereby configured to be mountable on different embodiments of laryngoscope blade **402,** differing from one another in the shape of blade superior surface **416.** In some embodiments laryngoscope blade **402** and guiding assembly **406** may be integrally formed, or, for example, tubular member **434** may be glued onto blade superior surface **416.** In some embodiments, wherein guiding assembly **406** includes a ratchet mechanism, as elaborated on below, when guiding assembly **406** is mounted on laryngoscope blade **402,** member proximal portion **462** may be superiorly elevated relative to blade superior surface **416** at blade proximal portion **412** in order to accommodate the ratchet mechanism. Loop **492** includes an arched portion **1472.** Arched portion **1472** is similar to expanded arch **132a,** being substantially shaped as a horseshoe and extending superiorly relative to blade superior surface **416** at blade distal portion **414** (when guiding assembly **406** is mounted on laryngoscope blade **402**). Arched portion **1472** includes an arched portion first arm **1474** and an arched portion second arm **1476.** Arched portion first arm **1474** consists of a portion of loop first arm **1412,** which extends from the inferior end of first spring coil **486** to loop top **1418.** Arched portion second arm **1476** consists of a portion of loop second arm **1414,** which extends from the inferior end of second spring coil **488** to loop top **1418.**

Similarly to looped element **104,** looped element **436** is switchable from a first configuration to a second configuration. In the first configuration, arched portion **1472** defines an expanded arch (shown in **Fig. 4A** wherein arched portion **1472** is depicted as fully expanded) similar to expanded arch **132a.** In the second configuration, arched portion **1472** defines a contracted arch (not shown) similar to contracted arch **132b.** Further, looped element **436** is continuously switchable from the first configuration to the second configuration, defining a range of mid-configurations with increasingly smaller arches, and from the second configuration to the first configuration, as elaborated on below. In the first configuration, spring **466** is relaxed, while in the second configuration spring **466** is compressed.

Spring coils **486** and **488** help sustain the superior extension of the arches from blade distal portion **414** (when guiding assembly **406** is mounted on laryngoscope blade **402**). In particular, spring coils **486** and **488** help maintain arched portion **1472** superior and lateral extension relative to blade distal portion **414** by supporting and directing arched portion first arm **1474** and arched portion second arm **1476,** respectively (which pass through spring coils **486** and **488,** respectively). It is noted that spring coils **486** and **488** are flexible, thereby facilitating safely accommodating different epiglottic and laryngopharyngeal architectures, which may greatly vary from one subject to another. In some embodiments, the spring coils can be coated by a non-stick material such as Teflon^{™}. In some embodiments, the spring coils can be covered by a PTFE coated sleeve.

The pivoting motion allows switching between a first handle configuration, shown in **Fig. 4A****,** and a second handle configuration (not shown). In the second handle configuration pivot angle **γ** is smaller than in the first handle configuration. For example, in the first configuration pivot angle **γ** may be about 30° and in the second configuration pivot angle **γ** may be about 10°. Further, in the second handle configuration, handle superior portion edge **1432** is positioned farther from member proximal tip **468** than in the first handle configuration, being substantially proximally shifted, or proximally and inferiorly shifted, relative to a position thereof in the first handle configuration. In some embodiments, in the first handle configuration, handle superior portion **448** is substantially parallel to mounting bar **444.**

In the first handle configuration, looped element **436** is in the first configuration. In the second handle configuration, looped element **436** is in the second configuration. To switch from the first handle configuration to the second handle configuration, elongated portion **446** is fully pressed towards mounting bar **444** and pivot angle **γ** is decreased. The resultant proximal pulling of handle superior portion edge **1432** results in an increase in the length of wire first segment **1452,** since elongated wire **490** is non-stretchable, in wire third segment **1456** being proximally pulled to compensate for the increase in wire first segment **1452** length (wire second segment **1454** length remains substantially unchanged). Wire third segment **1456** proximally pulls disc **1406,** which in turn compresses spring **466** by pushing spring **466** in the proximal direction. Further, disc **1406** proximally pulls distal wire portion **1404,** which in turn proximally pulls loop base **1416** into tubular member **434.** Consequently, loop base **1416** proximally pulls loop first arm **1412** and loop second arm **1414,** thereby shortening arched portion first arm **1474** and arched portion second arm **1476,** respectively. Arched portion first arm **1474** and arched portion second arm **1476** shortening results in the shrinking (i.e. contraction) of arched portion **1472,** thereby bringing looped element **436** into the second configuration (or into one of the mid-configurations if handle elongate portion **446** is not fully pressed).

In operation, laryngoscope **400** is inserted into oral cavity **208,** such that blade distal portion **414** is inserted into vallecula **224,** with arched portion **1472** encompassing epiglottis **226.** An elongated member, such as bougie introducer **206,** is next introduced into oral cavity **208** and distally advanced until a distal tip of the elongated member has passed through arched portion **1472** and is positioned proximally relative to corniculate tubercle **252,** essentially as described in the description of **Fig. 2A****.** Looped element **436** is then switched from the first configuration to the second configuration, or to one of the mid-configurations, and arched portion **1472** is contracted, as described above. The elongated member is then distally advanced farther, being directed by the contracted arch (or by an intermediate arch defined by one of the mid-configurations when handle elongate portion **446** is not fully pressed) into laryngeal inlet **262,** essentially as described above in the description of **Fig. 2B****.**

In some embodiments, first slot **1462** and second slot **1464** are elongated, extending along the length of blade distal portion **414,** such that when mounted thereon, connector first leg **482** and connector second leg **484** occupy only a portion along the respective lengths of first slot **1462** and second slot **1464.** For example, each of connector legs **482** and **484** may occupy a half, a third, a fifth, or even a tenth of the lengths of slots **1462** and **1464,** respectively. Further, slots **1462** and **1464** are configured to allow for connector **470** to be shifted along blade superior surface **416** when connector **470** is snap-engaged thereto, as elaborated on below.

A toothed wheel **1482** is mounted on mounting bar superior portion **454** between, and in parallel to, connecting bars **458.** Member proximal portion **462** includes teeth **1484** evenly disposed along the length of tubular member **434** on an inferior portion thereof (not numbered). Blade proximal portion **412** includes a blade slit **1486** extending from housing slot **430** in the distal direction. Elongated housing **428** includes a pair of housing slits: a housing first slit **1488** and a housing second slit **1489.** Housing slits **1488** and **1489** extend in the inferior direction on a housing proximal wall **1490** and on a housing distal wall **1492,** respectively, from housing superior end **1448.** A length of blade slit **1486** may be between about 1 cm to about 2 cm. Lengths of housing slits **1488** and **1489** may be between about 1 cm to about 3 cm.

Slits **1486, 1488,** and **1489** are configured to accommodate toothed wheel **1482** and teeth **1484** when guiding assembly **406** is mounted on laryngoscope blade **402.** Teeth **1484** are disposed superiorly to blade slit **1486** when guiding assembly **406** is mounted on laryngoscope blade **402.** Teeth **1484** and toothed wheel **1482** are configured for a ratchet-like engagement, thereby facilitating pulling tubular member **434** in the proximal direction (while maintaining hand grip **432** and laryngoscope blade **402** stationary). In some embodiments, the ratchet-like engagement is reversible, that is to say, two-way, thereby additionally facilitating pushing tubular member **434** in the distal direction. Toothed wheel **1482** may be turned, using, for example, a lever (not shown) mounted thereon.

An arched portion base **1405** is defined by the inferior end of arched portion first arm **1474** and the inferior end of arched portion second arm **1476.** A mounting site **1415** is defined by a segment of blade first edge **422** adjacent to first slot **1464** and by a segment of blade second edge **424** adjacent to second slot **1466.** A mounting mechanism is provided by first spring coil **486** and second spring coil **488.** The mounting mechanism (together with elongated wire **490** and hook **1442**) substantially restricts arched portion base **1405** to mounting site **1415** when connector **470** is snap-engaged to slots **1464** and **1466.**

In embodiments wherein laryngoscope **400** includes wheel **1482** and teeth **1484,** and slots **1464** and **1466** are elongated, mounting site **1415** is defined by a segment of blade first edge **422,** adjacent to the distal end of first slot **1464,** and by a segment of blade second edge **424,** adjacent to the distal end of second slot **1466.** A second mounting site **1435** is defined by a segment of blade first edge **422,** adjacent to the proximal end of first slot **1464,** and by a segment of blade second edge **424,** adjacent to the proximal end of second slot **1466.** By turning toothed wheel **1482,** and thereby proximally shifting tubular member **434,** arched portion base **1405** can be controllably shifted from mounting site **1415** to second mounting site **1435** (and vice-versa by turning toothed wheel **1484** in the opposite sense), and in some embodiments to additional mounting sites (not numbered) there between.

In some embodiments, hand grip **432** further includes a fourth spring **1494.** Fourth spring **1494** is mounted between mounting bar **444** and handle elongate portion **446,** being attached on one end thereof to mounting bar **444** and on the second end thereof to handle elongate portion **446.** Fourth spring **1494** acts to provide additional resistance (beyond that provided by spring **466**) to pressing handle elongate portion **446,** thereby in some embodiments providing increased control in switching looped element **436** from the first configuration to the mid-configurations. In embodiments including fourth spring **1494,** housing slit **1488** length may be about 5 cm, about 8 cm, or even about 10 cm or longer, in order to accommodate fourth spring **1494** (as well as toothed wheel **1482**). In some embodiments, housing slit **1488** may extend from housing superior end **1448** to housing inferior end **1446.**

According to some embodiments, tubular member **434** can be magnetically adhered to blade superior **416.** For example, tubular member **434** may have magnets (not shown) disposed on the inferior surface thereof. In such embodiments, elongated wire **490** and loop **492** may be made of plastic.

According to some embodiments, tubular member **434** is mounted on, and disposed along, blade inferior surface **418,** with loop **492** encompassing blade distal portion **414.** In such embodiments, first spring coil **486** and second spring coil **488** may be mounted on blade first edge **422** and blade second edge **424,** respectively.

Making reference again to **Fig. 3****,** in some embodiments laryngoscopy device **300** includes a pair of spring coils, such as spring coils **486** and **488,** mounted on blade superior surface **316** at blade distal portion **314** similarly to the mounting of spring coils **486** and **488** on connector **470.**

**Fig. 5** schematically depicts a laryngoscopy device **500,** including a laryngoscope blade **502** and a looped element **504,** according to some embodiments of the present disclosure. Laryngoscopy device **500** provides a specific embodiment of laryngoscopy device **100.** Laryngoscope blade **502** is elongated and includes a blade proximal portion **512** and a blade distal portion **514** (per the directions defined in **Fig. 1** which also apply for **Fig. 5**). Laryngoscope blade **502** further includes a blade superior surface **516,** a blade inferior surface **518,** and opposite lateral edges: a blade first edge **520,** and a blade second edge **522.** A blade tip **524** is defined by the distal edge of blade distal portion **514.** According to some embodiments, laryngoscope blade **502** may be mounted on a handle, such as handle **210.**

A length of laryngoscope blade **502** is indicated by a line **L6,** which extends from the proximal edge (not numbered) of blade proximal portion **512** to blade tip **524,** midway in between blade edges **520** and **522.**

Blade distal portion **514** includes a pair of parallel rails: a first rail **532** and a second rail **534.** Each of first rail **532** and second rail **534** is shaped as a narrow and elongated bar. First rail **532** proximally extends from blade tip **524** along blade distal portion **514,** in parallel to and proximately to blade first edge **520.** First rail **532** is elevated relative to blade superior surface **516,** e.g. by about 0.3 cm, and is joined thereto by first rail legs **536.** First rail legs **536** are positioned at the two ends (not numbered) of first rail **532,** projecting in the superior direction relative to blade superior surface **516.** Similarly, second rail **534** proximally extends from blade tip **524** along blade distal portion **514,** in parallel to and proximately to blade second edge **522.** Second rail **534** is elevated relative to blade superior surface **516,** e.g. by about 0.3 cm, and is joined thereto by second rail legs **538.** Second rail legs **538** are positioned at the two ends (not numbered) of second rail **534,** projecting in the superior direction relative to blade superior surface **516.** In some embodiments, first rail **532** and second rail **534** are between about 2 cm and about 6 cm long, or between about 3 cm to about 5 cm long. First rail **532** and second **rail 534** functionality is elaborated on below.

Blade tip **524** includes a canal **526,** in the form of an elongated passage, extending between two opposite openings: a canal first opening **528,** at blade first edge **520,** and a canal second opening **530** at blade second edge **522.** Blade tip **524** further includes a bore (not shown), bisecting canal **526** and pointing in the distal direction. A first push-button **542** is located on blade tip **524,** extending into blade tip **524** bore. A spring (not shown) is positioned within canal **526.** Canal **526** spring is mechanically connected to first push-button **542,** such that when canal **526** spring is relaxed, first push-button **542** projects outside of blade tip **524** in the distal direction (in addition to proximally extending into blade tip **524** bore). When first push-button **542** is pressed, first push-button **542** is proximally pushed into blade tip **524** bore and canal **526** spring is compressed. When the pressing is ceased, canal **526** spring pushes back first push-button **542** (i.e. in the distal direction).

First push-button **542** includes a hole (not shown). When first push-button **542** is pressed, first-push button **542** hole is aligned with canal **526.** When first push-button **542** is not pressed, first-push button **542** hole is only partially aligned with canal **526.** First push-button **542** functionality is further elaborated on below.

A second push-button **544** is located on blade inferior surface **518** at blade distal portion **514.** A stopper **546** is located oppositely to second push-button **544** on blade superior surface **516.** Stopper **546** includes a frame mount **552** and a stopper frame **554** mounted thereon (i.e. stopper frame **554** is elevated relative to blade superior surface **516**). In some embodiments, stopper frame **554** is shaped substantially as a square bracket (with rounded corners) and is mounted transversely relative to laryngoscope blade **502** length (i.e. transversely to line **L6**). In some embodiments, stopper frame **554** includes two pairs of frame legs: a first pair of legs **556** and a second pair of legs **558,** which are positioned proximately to blade first edge **520** and to blade second edge **522,** respectively, and which project in the inferior direction from stopper frame **554** towards blade superior surface **516.** The two legs of first pair of legs **556** are positioned on the two sides of first rail **532.** Similarly, the two legs of second pair of legs **558** are positioned on the two sides of second rail **534.**

Stopper **546** is mechanically associated with second push-button **544,** such that when second push-button **544** is pressed (i.e. pushed superiorly), stopper **546** is pushed in the superior direction. For example, in some embodiments second-push button **544** includes a spring (not shown) mounted perpendicularly to blade inferior surface **518.** Blade distal portion **514** includes a bore (not shown). Frame mount **552** extends through blade distal portion **514** bore and the inferior end (not shown) of frame-mount **552** is attached to second push-button **544** spring. In some embodiments second push-button **544** does not include a spring, but is made of an elastic material, such that when pressed, second push-button **544** superiorly pushes frame mount **552** (which extends through blade distal portion **514** bore and which is attached to second push-button **544**). Second push-button **544** and stopper **546** functionalities are further elaborated on below.

A first spring **562** and a second spring **564** are mounted on first rail **532** and second rail **534,** respectively. In some embodiments, both first spring **562** and second spring **564** are coil springs. First spring **562** longitudinally extends from a first spring distal end **572** to a first spring proximal end **574.** First spring distal end **572** is fixed proximately to blade tip **524.** For example, first spring distal end **572** may be attached (e.g. glued or welded) to the distal leg of the two legs constituting first rail legs **536.** Similarly, second spring **564** longitudinally extends from a second spring distal end **576** to a second spring proximal end **578.** Second spring distal end **576** is fixed proximately to blade tip **524.** First spring **562** has a relaxation length (i.e. a length thereof when not compressed or stretched) longer than first rail **532,** e.g. 9 cm when first rail **532** is 6 cm long. Similarly, second spring **564** has a relaxation length longer than second rail **534.**

In **Fig. 5** first spring **562** and second spring **564** are shown proximally extending until first pair of legs **556** and second pair of legs **558,** respectively. Both first spring **562** and second spring **564** are compressed (e.g. measuring 3 cm long). First pair of legs **556** and second pair of legs **558** bar first spring proximal end **574** and second spring proximal end **578** from proximally advancing along first rail **532** and second rail **534,** respectively. When second-push button **544** is pressed, stopper frame **554** and first pair of legs **556** and second pair of legs **558** are lifted, that is to say superiorly pushed away from blade superior surface **516.** Consequently, first spring proximal end **574** and second spring proximal end **578** are no longer barred by first pair of legs **556** and second pair of legs **558,** respectively, and move proximally along first rail **532** and second rail **534,** until barred from further proximal advance by the proximal leg of first rail legs **536** and the proximal leg of second rail legs **538,** respectively. First spring **562** and second spring **564** functionality is further elaborated on below.

Looped element **504** includes an elongated wire **580.** Elongated wire **580** extends from a wire first end **582** to a wire second end **584,** and includes a wire first portion **588,** a wire second portion **590,** and a wire arched portion **592.** Wire first portion **588** includes wire first end **582.** Wire second portion **590** includes wire second end **584.**

Wire first end **582** and wire second end **584** are attached to first spring proximal end **574** and second spring proximal end **578,** respectively. Wire first portion **588** is defined as a portion of elongated wire **580,** which extends from first spring proximal end **574** to canal second opening **530.** Wire first portion **588** extends outside of first spring **562,** and passes through canal **526** (entering canal **526** via canal first opening **528** and passing through first push-button **542** hole). Wire second portion **590** is defined as a portion of elongated wire **580,** which extends from second spring proximal end **578** to canal first opening **528.** Wire second portion **590** extends outside of second spring **564,** and passes through canal **526** (entering canal **526** via canal second opening **530** and passing through first push-button **542** hole). In some embodiments, wire first portion **588** and wire second portion **590** pass through first spring **562** and second spring **564,** respectively. Wire first portion **588** is joined to wire arched portion **592** at canal second opening **530.** Wire second portion **590** is joined to wire arched portion **592** on canal first opening **528.** When first-push button **542** is not pressed, first push-button **542** hole is only partially aligned with canal **526,** thereby clamping (i.e. pressing) wire first portion **588** and wire second portion **590** between walls (not shown) of first push-button **542** hole and walls (not shown) of canal **526.** The clamping of wire first portion **588** and wire second portion **590** prevents any movement of wire first portion **588** and wire second portion **590** along canal **526** (similarly to a drawstring mechanism).

Wire arched portion **592** is pre-shaped, similarly to a horseshoe, but in some embodiments is additionally bent as elaborated on below. Wire arched portion **592** is made of a material(s) which is both rigid and flexible, similarly to a catheter guidewire, as elaborated on above in the descriptions of looped element **104** and looped element **304.** Wire arched portion **592** rigidity is sufficient to maintain the (pre-shaped) shape thereof, unless acted on with sufficiently large forces. Wire arched portion **592** flexibility ensures that wire arched portion **592** returns to the (pre-shaped) shape thereof, after having been made to change shape (e.g. bent), so long as the applied forces were not too large. In particular, wire arched portion **592** rigidity ensures that wire arched portion **592** maintains the (pre-shaped) shape thereof when looped element **504** is mounted on laryngoscope blade **502,** while wire arched portion **592** flexibility ensures that wire arched portion **592** changes shape when pressed against walls of oral cavity **208** or walls of laryngopharynx **234,** e.g. laryngopharyngeal posterior wall **232,** during ETI.

Wire arched portion **592** includes an arched portion first arm **1502** and an arched portion second arm **1504.** Arched portion first arm **1502** and arched portion second arm **1504** are joined at an arched portion top **1510.** Similarly to looped element **104,** looped element **504** is switchable from a first configuration, defining an expanded arch similar to expanded arch **132a,** to a second configuration, defining a contracted arch (not shown) similar to contracted arch **132b.** **Fig. 5** depicts the first configuration, that is to say, wire arched portion **592** is shown expanded. Arched portion top **1510** is positioned superiorly relative to blade superior surface **516.** Further, arched portion top **1510** is positioned proximally relative to blade tip **524.** In some embodiments, in the first configuration, arched portion top **1510** may be positioned distally relative to blade tip **524,** or neither distally nor proximally.

Arched portion first arm **1502** extends from canal second opening **530** - whereat arched portion first arm **1502** is joined to wire first portion **588** - to arched portion top **1510.** Similarly, arched portion second arm **1504** extends from canal first opening **528** - whereat arched portion second arm **1504** is joined to wire second portion **590** - to arched portion top **1510.** Arched portion first arm **1502** includes a first arm mid-point **1512** dividing arched portion first arm **1502** into two portions (not numbered), which in some embodiments may be substantially equally long. Arched portion second arm **1504** includes a second arm mid-point **1514** dividing arched portion second arm **1504** into two portions (not numbered), which in some embodiments may be substantially equally long. First arm mid-point **1512** is positioned laterally and superiorly relative to canal second opening **530.** Second arm mid-point **1514** is positioned laterally and superiorly relative to canal first opening **528.** In some embodiments, in the first configuration, first arm mid-point **1512** and second arm mid-point **1514** are positioned distally relative to blade tip **524,** while arched portion top **1510** is positioned proximally relative to blade tip **524.** That is to say, in some embodiments, in the first configuration, wire arched portion **592** is proximally curved/bent.

An arched portion base **1526** is defined by the inferior end of arched portion first arm **1502** (which is joined to wire first portion **588**) and the inferior end of arched portion second arm **1504** (which is joined to wire second portion **590**)**.** A mounting site **1530** is defined by canal first opening **528** and canal second opening **530.** A mounting mechanism is provided by canal **526** walls and first push-button **542.** The mounting mechanism (together with first spring **562** and second spring **564**) restricts arched portion base **1526** to mounting site **1530,** that is to say, the inferior end of arched portion first arm **1502** to canal second opening **530** and the inferior end of arched portion second arm **1504** to canal first opening **528.**

In operation, laryngoscope device **500** is inserted into oral cavity **208,** such that blade distal portion **514** is inserted into vallecula **224,** with wire arched portion **592** encompassing epiglottis **226.** An elongated member, such as bougie introducer **206,** is next introduced into oral cavity **208** and distally advanced until a distal tip of the elongated member has passed through wire arched portion **592** and is positioned proximally relative to corniculate tubercle **252,** essentially as described in the description of **Fig. 2A****.** Looped element **504** is then switched to the second configuration, thereby directing the elongated member into laryngeal inlet **262,** essentially as described in the description of **Fig. 2B****.**

To switch from the first configuration, depicted in **Fig. 5****,** to the second configuration, laryngoscope blade **502** is pushed in both the distal direction (against the distalmost point of vallecula **224**) and the anterior direction (against base of the tongue **238**)**.** The distal pushing of laryngoscope blade **502** causes first push-button **542** to be pressed, bringing first push-button **542** hole into alignment with canal **526,** with the result that wire first portion **588** and wire second portion **590** are no longer clamped between canal **526** walls and the walls of first push-button **542** hole walls. The anterior pushing of laryngoscope blade **502** causes second push-button **544** to be pressed. Consequently, stopper frame **554** is pushed in the posterior direction, releasing first spring **562** and second spring **564,** that is to say, first spring proximal end **574** and second spring proximal end **578** are no longer barred by first pair of legs **556** and second pair of legs **558,** respectively. Since wire first end **582** and wire second end **584** are attached to first spring proximal end **574** and second spring proximal end **578** (and since wire first portion **588** and wire second portion **590** are no longer clamped inside canal **526**), the release of first spring **562** and second spring **564** releases also wire first portion **588** and wire second portion **590.**

Since first spring **562** is compressed, particularly in the first configuration, and since the relaxation length of first spring **562** is longer than first rail **532,** first spring **562** partially decompresses until first spring proximal end **574** is barred from further proximal advance by the proximal leg of first rail legs **536.** Similarly, since second spring **564** is compressed, particularly in the first configuration, and since the relaxation length of second spring **564** is longer than second rail **534,** second spring **564** partially decompresses until second spring proximal end **578** is barred from further proximal advance by the proximal leg of second rail legs **538.**

Since wire first end **582** is attached to first spring proximal end **574** (and since first-push button **542** is pressed and wire first portion **588** is consequently not clamped), first spring **562** decompression results in wire first end **582** being (proximally) advanced together with first spring proximal end **574.** Similarly, since wire second end **584** is attached to second spring proximal end **578** (and since first-push button **542** is pressed and wire second portion **590** is consequently not clamped), second spring **564** decompression results in wire second end **584** being (proximally) advanced together with second spring proximal end **578.** Consequently, each of wire first portion **588** and wire second portion **590** is lengthened, and wire arched portion **592** shrinks (i.e. arched portion first arm **1502** and arched portion second arm **1504** are shortened), thereby bringing looped element **504** into the second configuration.

In some embodiments, due to looped element **504** pre-shaping, in the second configuration, arched portion top **1510** is not proximally positioned relative to blade tip **524,** and may even be positioned distally relative thereto. Such pre-shaping of looped element **504** may facilitate insertion of bougie introducer **206** during ETI.

In some embodiments, laryngoscopy device **500** includes a flexible rod, such as flexible rod **340.** The flexible rod is connected at a distal tip thereof, such as rod tip **382,** to looped element **504,** e.g. at arched portion top **1510.** By distally pushing and proximally pulling the flexible rod, wire arched portion **592** shape and an orientation thereof may be adjusted, essentially as described in the description of **Fig. 3****.** Further, the flexible rod may be used to help switch from the second configuration back to the first configuration. To do so, both first push-button **542** and second push-button **544** are pressed, to allow wire first end **582** and wire second end **584** to be distally pulled. By proximally pulling the flexible rod, arched portion top **1510** is proximally pulled, thereby expanding wire arched portion **592** and bringing looped element **504** into the first configuration. In particular, arched portion first arm **1502** and arched portion second arm **1504** are pulled with the result that first spring proximal end **574** and second spring proximal end **578** are proximally pulled and first spring **562** and second spring **564** are compressed. First push-button **542** and second push-button **544** are then released. Consequently, wire first portion **588** and wire second portion **590** become clamped in canal **526,** and, further, first pair of legs **556** and second pair of legs **558** are pushed towards blade superior surface **516,** thereby barring first spring proximal end **574** and second spring proximal end **578,** respectively (and securing looped element **504** in the first configuration).

According to some embodiments, a third arm **1532** and a fourth arm **1534** extend from, or from about, first arm mid-point **1512** and second arm mid-point **1514,** respectively. A second arched portion **1540** (marked by a dashed line) is formed by third arm **1532** and fourth arm **1534,** which are joined at a second arched portion top **1542.** Second arched portion top **1542** is positioned superiorly relative to blade superior surface **516** and proximally relative to arched portion top **1510.** In some embodiments, second arched portion **1540** is connected to wire arched portion **592** by e.g. a thin metal net or plastic film (which may be collapsible or flexible). Second arched portion **1540** may help in guiding bougie introducer **206** (or other types of elongated intubation member, such an endotracheal tube) into laryngeal inlet **262** during ETI, by providing better support and the possibility of angulation.

It is noted that a guiding instrument, resembling laryngoscopy device **100,** may be used for increasing control and accuracy in minimally invasive procedures. In some embodiments, the guiding instrument is longer than laryngoscopy device **100,** for example, the guiding instrument can be about 20 cm long, about 30 cm long, or even about 50 cm long. In some embodiments, the guiding instrument may be mounted on (e.g. glued to) a laparoscopic or thoracoscopic trocar or an endoscope at the respective distal ends thereof, such that an arch (similar to expanded arch **132a**) defined by a looped element (similar to looped element **104**) of the guiding instrument projects away from the guiding instrument. In some embodiments, the guiding instrument can be flexible and attached to a flexible endoscope. To controllably maneuver a device (e.g. a grasping device, a surgical stapler, or a fiber-optic light source) proximately to the laparoscopic trocar or the endoscope, the laparoscopic trocar or the endoscope, with the guiding instrument mounted thereon, as described above, may be inserted into a hollow cavity (e.g. stomach, bowel, abdomen, urinary bladder, uterus, ear canal, nostril, etc.). Next, the device may be advanced from the site of insertion (of the laparoscopic trocar or the endoscope), or from a different site, until a distal tip of the device passes through the arch. The looped element is then switched from the first configuration to the second configuration. The device may have projecting elements that can be hooked to matching elements on the guiding instrument, enabling a secured connection there between.

According to an aspect of some embodiments, there is provided a laryngoscopy device (e.g. **100, 204, 300, 400, 500**) including:
- a laryngoscope blade (e.g. **102, 302, 402, 502**), being elongated, having a blade distal portion (e.g. **114, 314, 414, 514**), a blade first edge (e.g. **122, 322, 422, 520**), and a blade second edge (e.g. **124, 324, 424, 522**), opposite to the blade first edge; and
- a looped element (e.g. **104, 304, 436, 504**), switchable at least from a first configuration to a second configuration.

In the first configuration and in the second configuration the looped element defines an expanded arch (e.g. **132a, 1472, 592**) and a contracted arch (e.g. **132b**), respectively. The expanded arch has an expanded arch first arm (e.g. **134a, 1474**) and an expanded arch second arm (e.g. **136a, 1476**), joined at an expanded arch top (e.g. **138a, 1418, 1510**)**.** The contracted arch has a contracted arch first arm (e.g. **134b**) and a contracted arch second arm (e.g. **136b**), joined at a contracted arch top (e.g. **138b**)**.**

The expanded arch top is positioned superiorly relative to the blade distal portion. The expanded arch first arm at least partially extends laterally from about the blade first edge and the expanded arch second arm at least partially extends laterally from about the blade second edge.

According to some embodiments, the looped element (e.g. **104, 304, 436**) is continuously switchable from the first configuration to the second configuration, across a range of mid-configurations, defining a respective range of increasingly smaller arches (e.g. **332**), ranging from the expanded arch to the contracted arch.

According to some embodiments, the looped element (e.g. **104, 304, 436, 504**) is additionally switchable from the second configuration to the first configuration.

According to some embodiments, the looped element (e.g. **104, 304, 436**) is continuously switchable from the second configuration to the first configuration. According to some embodiments, the contracted arch top (e.g. **138b**) is positioned superiorly relative to the blade distal portion.

According to some embodiments, the blade distal portion is curved and the expanded arch (e.g. **132a, 1472, 592**) and the contracted arch (e.g. **132b**) are each configured such as (i) to encompass the epiglottis of a subject when the laryngoscopy device is inserted into the oral cavity of the subject with the blade distal portion being inserted into the vallecula of the subject, and (ii) to simultaneously facilitate guiding of an elongated intubation member through the expanded arch and through the contracted arch, respectively.

According to some embodiments, the elongated intubation member is a bougie introducer (e.g. **206**)**,** a stylet, or an endotracheal tube.

According to some embodiments, there is provided a laryngoscope including the laryngoscopy device and a handle (e.g. **210, 432**), wherein the laryngoscope blade is mechanically connected to the handle.

According to an aspect of some embodiments, there is provided a laryngoscopy device (e.g. **100, 204, 400, 500**) including:
- a laryngoscope blade (e.g. **102, 402, 502**), being elongated, having a blade distal portion (e.g. **114, 414, 514**);
- a looped element (e.g. **104, 436, 504**), switchable at least from a first configuration to a second configuration; and
- a mounting mechanism (e.g. **486** and **488, 526** and **542**).

In the first configuration and in the second configuration the looped element respectively defines an expanded arch (e.g. **132a, 1472, 592**), having an expanded arch base (e.g. **550a, 1405, 1526**), and a contracted arch (e.g. **132b**), having a contracted arch base (e.g. **550b**).

The mounting mechanism supports (for example, mechanically supports) the expanded arch and the contracted arch in the first configuration and in the second configuration, respectively.

The mounting mechanism substantially restricts the expanded arch base and the contracted arch base to a mounting site (e.g. **170, 1415, 1530**) on the blade distal portion.

According to some embodiments, an arch top (e.g. **138a, 1418, 1510**) of the expanded arch is positioned superiorly relative to the blade distal portion.

According to some embodiments, the expanded arch base and the contracted arch base are controllably shiftable from the mounting site to at least one other mounting site (e.g. **1435**) on the blade distal portion.

According to some embodiments, the expanded arch is wider than the blade distal portion.

According to an aspect of some embodiments, there is provided a method for performing laryngoscopy on a subject. The method includes the steps of:
- providing any one of the laryngoscopy devices (e.g. **100, 204, 300, 400, 500**) and an elongated intubation member (e.g. **206**);
- introducing the laryngoscopy device into the oral cavity of the subject with the looped element being in the first configuration;
- maneuvering the laryngoscope blade such as to insert the blade distal portion into the vallecula of the subject, thereby encompassing the epiglottis of the subject with the expanded arch;
- introducing the elongated intubation member into the oral cavity and distally advancing the elongated intubation member until a member distal tip thereof passes through the expanded arch and is proximally positioned relative to the corniculate tubercle of the subject;
- switching from the first configuration to the second configuration thereby directing the member distal tip towards the laryngeal inlet of the subject; and
- distally advancing the member distal tip into the laryngeal inlet of the subject.

According to some embodiments, the elongated intubation member is a bougie introducer (e.g. **206**), and the method further includes, following the step of distally advancing the member distal tip into the laryngeal inlet of the subject, a step of mounting an endotracheal tube on the bougie introducer.

According to some embodiments, the elongated intubation member is an endotracheal tube.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" or "comprising," when used in this specification, specify the presence of stated features, integers, steps, operations, elements, or components, but do not preclude or rule out the presence or addition of one or more other features, integers, steps, operations, elements, components, or groups thereof.

While a number of exemplary aspects and embodiments have been discussed above, those of skill in the art will recognize certain modifications, additions and sub-combinations thereof. It is therefore intended that the following appended claims and claims hereafter introduced be interpreted to include all such modifications, additions and sub-combinations.

## Claims

1. A laryngoscopy device (100) comprising:
a laryngoscope blade (102), being elongated, having a blade distal portion (114) comprising a blade tip (126);
a looped element (104), switchable at least from a first configuration to a second configuration; and
a mounting mechanism;
wherein in the first configuration said looped element (104) defines an expanded arch (132a), having an expanded arch base (150a) and an expanded arch top (138a), and in the second configuration said looped element (104) defines a contracted arch (132b), having a contracted arch base (150b);
wherein said mounting mechanism (486 and 488, 526 and 542) is configured to support said expanded arch (132a) and said contracted arch (132b) in the first configuration and in the second configuration, respectively;
wherein said mounting mechanism (486 and 488, 526 and 542) is configured to restrict said expanded arch base (150a) and said contracted arch base (150b) to a first mounting site (170, 1415) on said blade distal portion (114); and
**characterized in that** said expanded arch (132a) is shaped and sized such that when said laryngoscopy device (100) is inserted into the oral cavity of the subject in said first configuration with said blade tip (126) being located in a vallecula of the subject and said looped element (104) encompassing the epiglottis, then said expanded arch top (138a) is positioned proximately to, or rests against, the laryngopharyngeal posterior wall.

2. The laryngoscopy device (100) of claim 1 wherein said expanded arch base (150a) comprises an expanded arch first arm (134a) and an expanded arch second arm (136a), and wherein said looped element (104) is configured such that to switch from the first configuration to the second configuration said expanded arch first arm (134a) and/or said expanded arch second arm (136a) is pulled.

3. The laryngoscopy device (100) of any one of claims 1 and 2 wherein said contracted arch (132b) further comprises a contracted arch top (138b) , and wherein said contracted arch (132b) is shaped and sized such that said contracted arch top (138b) is configured to be positioned more proximately to an epiglottis of the subject than to the posterior laryngopharyngeal wall, when said looped element (104) is in the second configuration and said laryngoscope blade (102) is inserted into an oral cavity of the subject with said blade tip (126) being inserted into a vallecula of the subject and said looped element (104) encompassing the epiglottis.

4. The laryngoscope device (100) of claim 3 wherein a size of said contracted arch (132b) is such as to enable guiding by said contracted arch (132b) of an elongated intubation member into a laryngeal inlet of the subject, when said laryngoscope blade (102) is inserted into an oral cavity of the subject with said blade tip (126) being inserted into a vallecula of the subject and said looped element (104) encompassing the epiglottis.

5. The laryngoscopy device (100) of claim 4 wherein said elongated intubation member is a bougie introducer, a stylet, or an endotracheal tube.

6. The laryngoscopy device (100) of any one of claims 1 to 5 wherein said looped element (104) is flexible and resilient.

7. The laryngoscopy device (100) of claim 6 wherein said looped element (104) comprises a wire and is configured such that to switch from the first configuration to the second configuration said wire is pulled.

8. The laryngoscopy device (100) of any one of claims 6 and 7 wherein, by pulling said looped element (104), said looped element (104) is continuously switchable from the first configuration to the second configuration, across a range of mid-configurations, defining a respective range of increasingly smaller arches, ranging from said expanded arch (132a) to said contracted arch (132b).

9. The laryngoscopy device (100) of any one of claims 6 to 8 wherein, by pulling a top of said looped element (104) or by loosening said looped element (104), said looped element (104) is additionally switchable from the second configuration to the first configuration; preferably wherein said looped element (104) is continuously switchable from the second configuration to the first configuration.

10. The laryngoscopy device (100) of any one of claims 1 to 9 wherein said laryngoscope blade (102) is curved, such that, when said laryngoscope blade (102) is inserted into an oral cavity of a subject with said blade tip (126) being inserted into a vallecula of the subject and said looped element (104) encompassing the epiglottis, said blade distal portion (114) is curved in the anterior direction.

11. The laryngoscopy device (100) of any one of claims 1 to 10 wherein said expanded arch (132a) is wider than said blade distal portion (114).

12. The laryngoscopy device (100) of claim 11 wherein said expanded arch base (150a) and said contracted arch base (150b) are movable from said first mounting site (1415) to a second mounting (1435) site on said blade distal portion (114), wherein the second mounting site (1435) is longitudinally positioned proximally or distally relative to the first mounting site (1415).

13. A laryngoscope comprising the laryngoscopy device (100) of any one of claims 1 to 12 and a handle (210), wherein said laryngoscope blade (102) is connected to said handle (210).

## Patentansprüche

1. Laryngoskopievorrichtung (100), umfassend:
einen Laryngoskopspatel (102), der länglich ist und einen distalen Spatelabschnitt (114) hat, der eine Spatelspitze (126) umfasst,
ein schlaufenförmiges Element (104), das mindestens aus einer ersten Konfiguration in eine zweite Konfiguration wechseln kann, und
einen Montagemechanismus,
wobei das schlaufenförmige Element (104) in der ersten Konfiguration einen expandierten Bogen (132a) mit einer expandierten Bogenbasis (150a) und einer expandierten Bogenoberseite (138a) definiert, und das schlaufenförmige Element (104) in der zweiten Konfiguration einen zusammengezogenen Bogen (132b) mit einer zusammengezogenen Bogenbasis (150b) definiert,
wobei der Montagemechanismus (486 und 488, 526 und 542) dazu ausgestaltet ist, den expandierten Bogen (132a) und den zusammengezogenen Bogen (132b) jeweils in der ersten Konfiguration und der zweiten Konfiguration zu stützen,
wobei der Montagemechanismus (486 und 488, 526 und 542) dazu ausgestaltet ist, die expandierte Bogenbasis (150a) und die zusammengezogene Bogenbasis (150b) auf eine erste Montagestelle (170, 1415) an dem distalen Spatelabschnitt (114) zu beschränken, und
**dadurch gekennzeichnet, dass** der expandierte Bogen (132a) so geformt und bemessen ist, dass die expandierte Bogenoberseite (138a) in der Nähe der laryngopharyngealen Rückwand positioniert ist oder daran anliegt, wenn die Laryngoskopievorrichtung (100) in der ersten Konfiguration, in der die Spatelspitze (126) in einer Vallecula des Subjekts angeordnet ist und das schlaufenförmige Element (104) die Epiglottis umschließt, in die Mundhöhle des Subjekts eingeführt ist.

2. Laryngoskopievorrichtung (100) nach Anspruch 1, wobei die expandierte Bogenbasis (150a) einen ersten Arm (134a) des expandierten Bogens und einen zweiten Arm (136a) des expandierten Bogens umfasst und wobei das schlaufenförmige Element (104) so ausgestaltet ist, dass zum Wechseln aus der ersten Konfiguration in die zweite Konfiguration an dem ersten Arm (134a) des expandierten Bogens und/oder dem zweiten Arm (136a) des expandierten Bogens gezogen wird.

3. Laryngoskopievorrichtung (100) nach einem der Ansprüche 1 und 2, wobei der zusammengezogene Bogen (132b) ferner eine zusammengezogene Bogenoberseite (138b) umfasst und wobei der zusammengezogene Bogen (132b) so geformt und bemessen ist, dass die zusammengezogene Bogenoberseite (138b) so ausgestaltet ist, dass sie näher an der Epiglottis des Subjekts als der laryngopharyngealen Rückwand positioniert ist, wenn das schlaufenförmige Element (104) in der zweiten Konfiguration ist und der Laryngoskopspatel (102) in die Mundhöhle des Subjekts eingeführt ist, wobei die Spatelspitze (126) in eine Vallecula des Subjekts eingeführt ist und das schlaufenförmige Element (104) die Epiglottis umschließt.

4. Laryngoskopievorrichtung (100) nach Anspruch 3, wobei eine Größe des zusammengezogenen Bogens (132b) derart ist, dass ein längliches Intubationsglied mittels des zusammengezogenen Bogens (132b) in einen laryngealen Einlass des Subjekts hineingeführt werden kann, wenn der Laryngoskopspatel (102) in die Mundhöhle eines Subjekts eingeführt wird, wobei die Spatelspitze (126) in eine Vallecula des Subjekts eingeführt wird und das schlaufenförmige Element (104) die Epiglottis umschließt.

5. Laryngoskopievorrichtung (100) nach Anspruch 4, wobei das längliche Intubationsglied ein Bougie-Einführer, ein Stilett oder ein Endotrachealtubus ist.

6. Laryngoskopievorrichtung (100) nach einem der Ansprüche 1 bis 5, wobei das schlaufenförmige Element (104) flexibel und federnd ist.

7. Laryngoskopievorrichtung (100) nach Anspruch 6, wobei das schlaufenförmige Element (104) einen Draht umfasst und so ausgestaltet ist, dass es aus der ersten Konfiguration in die zweite Konfiguration wechselt, wenn an dem Draht gezogen wird.

8. Laryngoskopievorrichtung (100) nach einem der Ansprüche 6 und 7, wobei das schlaufenförmige Element (104) durch Ziehen an dem schlaufenförmigen Element (104) durchgehend über einen Bereich von mittleren Konfigurationen, die einen jeweiligen Bereich von immer kleiner werdenden Bögen von dem expandierten Bogen (132a) zu dem zusammengezogenen Bogen (132b) definiert, aus der ersten Konfiguration in die zweite Konfiguration wechseln kann.

9. Laryngoskopievorrichtung (100) nach einem der Ansprüche 6 bis 8, wobei das schlaufenförmige Element (104) zusätzlich aus der zweiten Konfiguration in die erste Konfiguration wechseln kann, indem an einer Oberseite des schlaufenförmigen Elements (104) gezogen wird oder indem das schlaufenförmige Element (104) gelockert wird, vorzugsweise wobei das schlaufenförmige Element (104) durchgehend aus der zweiten Konfiguration in die erste Konfiguration wechseln kann.

10. Laryngoskopievorrichtung (100) nach einem der Ansprüche 1 bis 9, wobei der Laryngoskopspatel (102) gekrümmt ist, so dass der distale Spatelabschnitt (114) in der vorderen Richtung gekrümmt ist, wenn der Laryngoskopspatel (102) in die Mundhöhle eines Subjekts eingeführt ist, wobei die Spatelspitze (126) in eine Vallecula des Subjekts eingeführt ist und das schlaufenförmige Element (104) die Epiglottis umschließt.

11. Laryngoskopievorrichtung (100) nach einem der Ansprüche 1 bis 10, wobei der expandierte Bogen (132a) weiter als der distale Spatelabschnitt (114) ist.

12. Laryngoskopievorrichtung (100) nach Anspruch 11, wobei die expandierte Bogenbasis (150a) und die zusammengezogene Bogenbasis (150b) von der ersten Montagestelle (1415) zu einer zweiten Montagestelle (1435) an dem distalen Spatelabschnitt (114) bewegbar ist, wobei die zweite Montagestelle (1435) in Längsrichtung proximal oder distal bezüglich der ersten Montagestelle (1415) positioniert ist.

13. Laryngoskop, umfassend die Laryngoskopievorrichtung (100) nach einem der Ansprüche 1 bis 12 und einen Griff (210), wobei der Laryngoskopspatel (102) mit dem Griff (210) verbunden ist.

## Revendications

1. Dispositif laryngoscopique (100) comprenant :
une lame (102) de laryngoscope, étant allongée, possédant une partie distale (114) de lame comprenant une pointe (126) de lame ;
un élément en boucle (104), pouvant passer au moins d'une première configuration à une seconde configuration ; et
un mécanisme de montage ;
dans lequel, dans la première configuration, ledit élément en boucle (104) définit un arc étendu (132a), possédant une base (150a) d'arc étendu et une partie supérieure (138a) d'arc étendu, et dans la seconde configuration, ledit élément en boucle (104) définit un arc contracté (132b), possédant une base (150b) d'arc contracté ;
dans lequel ledit mécanisme de montage (486 et 488, 526 et 542) est conçu pour supporter ledit arc étendu (132a) et ledit arc contracté (132b) dans la première configuration et dans la seconde configuration, respectivement ;
dans lequel ledit mécanisme de montage (486 et 488, 526 et 542) est conçu pour limiter ladite base (150a) d'arc étendu et ladite base (150b) d'arc contracté à un premier site de montage (170, 1415) sur ladite partie distale (114) de lame ; et
**caractérisé en ce que** ledit arc étendu (132a) est formé et dimensionné de sorte que, quand ledit dispositif laryngoscopique (100) est introduit dans la cavité buccale du sujet dans ladite première configuration avec ladite pointe (126) de lame située dans une vallécule du sujet et ledit élément en boucle (104) englobant l'épiglotte, ladite partie supérieure (138a) d'arc étendu soit positionnée à proximité de la paroi postérieure laryngopharyngée ou repose contre celle-ci.

2. Dispositif laryngoscopique (100) selon la revendication 1 dans lequel ladite base (150a) d'arc étendu comprend un premier bras (134a) d'arc étendu et un second bras (136a) d'arc étendu, et dans lequel ledit élément en boucle (104) est conçu de sorte que, pour passer de la première configuration à la seconde configuration, ledit premier bras (134a) d'arc étendu et/ou ledit second bras (136a) d'arc étendu soient tirés.

3. Dispositif laryngoscopique (100) selon l'une quelconque des revendications 1 et 2 dans lequel ledit arc contracté (132b) comprend en outre une partie supérieure (138b) d'arc contracté, et dans lequel ledit arc contracté (132b) est formé et dimensionné de sorte que ladite partie supérieure (138b) d'arc contracté soit conçue pour être positionnée plus près de l'épiglotte du sujet que de la paroi laryngopharyngée postérieure, lorsque ledit élément en boucle (104) est dans la seconde configuration et que ladite lame (102) de laryngoscope est introduite dans la cavité buccale du sujet avec ladite pointe (126) de lame insérée dans une vallécule du sujet et ledit élément en boucle (104) englobant l'épiglotte.

4. Dispositif laryngoscopique (100) selon la revendication 3 dans lequel une taille dudit arc contracté (132b) est telle qu'elle permet un guidage par ledit arc contracté (132b) d'un élément d'intubation allongé dans une entrée laryngée du sujet, lorsque ladite lame (102) de laryngoscope est introduite dans la cavité buccale du sujet avec ladite pointe (126) de lame insérée dans une vallécule du sujet et ledit élément en boucle (104) englobant l'épiglotte.

5. Dispositif laryngoscopique (100) selon la revendication 4 dans lequel ledit élément d'intubation allongé est un introducteur de bougie, un stylet ou un tube endotrachéal.

6. Dispositif laryngoscopique (100) selon l'une quelconque des revendications 1 à 5 dans lequel ledit élément en boucle (104) est flexible et résilient.

7. Dispositif laryngoscopique (100) selon la revendication 6 dans lequel ledit élément en boucle (104) comprend un fil et est conçu de sorte que, pour passer de la première configuration à la seconde configuration, ledit fil soit tiré.

8. Dispositif laryngoscopique (100) selon l'une quelconque des revendications 6 et 7 dans lequel, en tirant sur ledit élément en boucle (104), ledit élément en boucle (104) peut continuellement passer de la première configuration à la seconde configuration, sur une plage de configurations intermédiaires, définissant une plage respective d'arcs de plus en plus petits, allant dudit arc étendu (132a) audit arc contracté (132b).

9. Dispositif laryngoscopique (100) selon l'une quelconque des revendications 6 à 8 dans lequel, en tirant sur une partie supérieure dudit élément en boucle (104) ou en desserrant ledit élément en boucle (104), ledit élément en boucle (104) peut en outre passer de la seconde configuration à la première configuration ; de préférence dans lequel ledit élément en boucle (104) peut continuellement passer de la seconde configuration à la première configuration.

10. Dispositif laryngoscopique (100) selon l'une quelconque des revendications 1 à 9 dans lequel ladite lame (102) de laryngoscope est courbée, de sorte que, lorsque ladite lame (102) de laryngoscope est introduite dans la cavité buccale d'un sujet avec ladite pointe (126) de lame insérée dans une vallécule du sujet et ledit élément en boucle (104) englobant l'épiglotte, ladite partie distale (114) de lame soit courbée dans la direction antérieure.

11. Dispositif laryngoscopique (100) selon l'une quelconque des revendications 1 à 10 dans lequel ledit arc étendu (132a) est plus large que ladite partie distale (114) de lame.

12. Dispositif laryngoscopique (100) selon la revendication 11 dans lequel ladite base (150a) d'arc étendu et ladite base (150b) d'arc contracté sont mobiles depuis ledit premier site de montage (1415) vers un second site de montage (1435) sur ladite partie distale (114) de lame, dans lequel le second site de montage (1435) est positionné longitudinalement de façon proximale ou distale par rapport au premier site de montage (1415).

13. Laryngoscope comprenant le dispositif laryngoscopique (100) selon l'une quelconque des revendications 1 à 12 et un manche (210), dans lequel ladite lame (102) de laryngoscope est reliée audit manche (210).
